# EUROPEAN PATENT SPECIFICATION

(11) **EP 4 218 858 B1**
(45) Date of publication and mention of the grant of the patent: **21.10.2026**
(21) Application number: 20954567.2
(22) Date of filing: 25.09.2020
(51) Int. Cl.: A61M 5/20, A61M 5/14, A61M 5/142

(54) **INJECTOR**
INJEKTOR
INJECTEUR

(43) Date of publication of application: 02.08.2023
(60) Divisional application: 24167844.0 / 4 374 893
(73) Proprietor: C C Biotechnology Corporation, Tainan City, Taiwan 70955 (TW)
(72) Inventor: YEH, Chin-Min, Tainan City, Taiwan (CN); WU, Pin-Han, Tainan City, Taiwan (CN)
(74) Representative: Cabinet Chaillot
(86) International application number: PCT/CN2020/117746
(87) International publication number: WO 2022/061715

(56) References cited:
- CN-A- 101 495 165
- CN-A- 103 402 619
- CN-U- 207 804 718
- US-A1- 2008 097 375
- US-A1- 2012 118 138
- US-A1- 2018 228 972
- US-A1- 2019 209 773
- US-A1- 2020 171 242
- US-B2- 10 583 244

## Description

### Field of the Invention

The present invention relates to an injector used for subcutaneous injection of a pharmaceutical agent into a human body.

### Description of the Prior Arts

A conventional injector for subcutaneous injection of a pharmaceutical agent into a human body has a manually or electrically operated injection module connected to a medicament container, so a needle assembly of the injection module can pierce the human body for subcutaneous injections.

Due to safety regulations of medicament liquid injections, the conventional injector is limited in times of reuses, and once the number of times of using the conventional injector reaches the limit, the whole conventional injector should be abandoned and the conventional injector can no longer be reused. Additionally, the conventional injector is designed with a non-detachable structure, and a driving mechanism is directly connected to an injection module, thereby contacting the medicament liquid. Thus, for safety and health reasons, parts of the conventional injector cannot be detached and reused.

Besides, in order to prevent discomfort of the human body caused by piercing and removal of needle many times when multiple subcutaneous injections are to be performed on the human body, a needle assembly of the conventional injector has a soft needle and a hard needle. Since the soft needle is soft and thus is difficult to directly pierce the human body, the soft needle pierces the human body with the hard needle mounted inside the soft needle. Afterward, the hard needle is drawn out from the soft needle and the soft needle is connected to the injection module and the medicament container for injections. The soft needle is able to stay longer in the human body for multiple injections. However, in the injection module, since the needle assembly has the soft needle and the hard needle, an operator has to set up a needle with two steps, which is inconvenient and needs to be further improved.

A conventional FLUID DELIVERY SYSTEM COMPRISING A FLUID PUMPING DEVICE AND A DRIVE SYSTEM, as disclosed in US 2012/0118138 A1, discloses a fluid pumping device having a pump housing and a valve switching element. The pumping housing has a piston chamber, a reciprocating piston, an inlet port and an outlet port, and allows a fluid to enter the piston chamber during an instroke of the piston and be expelled during an outstroke. The valve switching element is movably mounted on a valve base member, has a piston chamber aperture connected to the piston chamber, and has an inlet aperture and an outlet aperture connected respectively to the inlet and outlet ports. A groove in the valve base member provides a first communication between the inlet aperture and the piston chamber aperture so that the fluid is sucked into the piston chamber during part of the piston instroke, and a second communication between the piston chamber aperture and the outlet aperture so that the fluid is expelled out of the piston chamber through the outlet port during part of the piston outstroke.

A conventional INFUSION PUMPS AND METHODS AND DELIVERY DEVICES AND METHODS WITH THE SAME, as disclosed in US 2008/097375 A1, discloses a delivery device including a durable housing portion and a separable disposable portion that selectively engage and disengage from each other. The disposable housing portion is configured to secure to a patient-user and may be disposed of after having been in use for a prescribed period. Components that normally come into contact with the patient-user or with an infusion medium are supported by the disposable housing portion for disposal after the prescribed use. The durable housing portion supports components that do not come into contact with the infusion medium or the patient-user during normal operation of the delivery device.

The main objective of the present invention is to provide an injector that solves the problem that the conventional injector should be abandoned after used due to safety concern.

The injector has a medicament container, an injection module, and a driving mechanism. The medicament container has a container main body accommodating medicament and a container connecting port mounted on a side of the container main body. The injection module has a conversion mechanism and a needle output mechanism. The conversion mechanism is connected to the medicament container. The conversion mechanism has a conversion connecting base and a driving piston set mounted in the conversion connecting base. The conversion connecting base has an input adapter and an output adapter. The conversion connecting base is connected to the container connecting port of the medicament container via the input adapter. The needle output mechanism is connected to the conversion mechanism. The needle output mechanism has a needle set. The needle set is capable of passing the output adapter. The driving mechanism is detachably connected to the conversion mechanism of the injection module. The driving mechanism has a case and a driving unit mounted in the case and adapted to be operated to move. The case is detachably connected to the conversion connecting base of the conversion mechanism. The driving unit is connected to the driving piston set and is capable of driving the driving piston set to draw and transport medicament from the medicament container to the needle output mechanism, and then injecting the medicament with the needle set of the needle output mechanism.

The needle output mechanism has a base, a connecting member, a needle set, a needle output operating assembly, and a needle returning operating unit. The connecting member is moveably mounted in the base. The needle set is moveably mounted in the connecting member and has a soft needle, a needle plug, and a hard needle. The needle plug is mounted on an end of the soft needle. The hard needle is capable of being mounted in the soft needle. The needle output operating assembly is moveably mounted in the base and is connected to the needle set. The needle output operating assembly is capable of being operated to drive the needle set located in the connecting member to protrude out of the base with the hard needle mounted in the soft needle, and then drive the hard needle to detach from the soft needle. The needle returning operating unit is moveably mounted in the base. The needle returning operating unit is capable of being operated to drive the connecting member and the soft needle in a needle-output state to retract back to the base.

The advantages of the present invention are as follows. The injection module has a conversion mechanism and a needle output mechanism connected to each other. The conversion mechanism is detachably connected to the medicament container. A driving piston set of the conversion mechanism is detachably connected to the driving mechanism. The driving mechanism is capable of driving the driving piston set to transport medicament in the medicament container to the needle set for injection. With the aforementioned modular construction, the medicament container and the injection module of the injector relating to safety and hygiene is single-use and can be detached and abandoned after used. Besides, the driving mechanism is mounted on the conversion mechanism, so the driving mechanism will not contact medicament during injection and thus can be reused, thereby reducing unnecessary wasting.

During injection to a human body by the injector, the protruding operating assembly is operated to drive the needle set located in the connecting member, and makes the needle set protrude from the base with the hard needle mounted in the soft needle, such that the soft needle of the needle set is capable of piercing into the human body. During the operation applied on the needle output operating assembly, the hard needle is then driven to detach from the soft needle, and then the drive module drives the driving piston assembly of the adapter mechanism to run pharmaceutical agent injection. After injection, the needle returning operating unit is operated to retract the soft needle. As a result, the needle output mechanism of the present invention facilitates ease in operation.

Further, the base has an action room. A back surface of the base has a base board and a penetration hole formed on the base board and connected to the action room. A side of the base has an action opening connected to the action room. A fixing buckling hook is mounted on the base and is located outside the action room. The connecting member is moveably mounted in the action room of the base. The connecting member has a connecting base segment and a connecting head. The connecting base segment has a base annular wall and a needle set mounting segment mounted in the base annular wall. The needle set mounting segment has a needle room formed in the needle set mounting segment and a needle plug hole formed on an end, which faces toward the base board, of the needle set mounting segment and connected to the needle room. The needle plug hole corresponds to the penetration hole of the base board. The connecting head is mounted on the connecting base and is connected to the needle room in the needle set mounting segment. The connecting head is mounted through the action opening of the base. The base annular wall has at least one cutting blade segment. The soft needle of the needle set is moveably mounted in the needle room of the needle set mounting segment. The needle plug that is mounted on the soft needle is capable of being fixed in the needle plug hole. The needle output operating assembly has a needle output operating unit, a needle base, a sealing unit, and an elastic unit. The needle output operating unit is moveably mounted in the base, and the needle output operating unit is capable of being fixed and buckled by the fixing buckling hook of the base. The needle output operating unit has a button segment and an accommodating room located on a side of the button segment. The accommodating room corresponds to the action room. The needle base is located in the accommodating room of the needle output operating unit and is connected to the needle output operating unit. The needle base has a needle base head end and a needle base connecting rod. A connecting segment is mounted between a periphery of the needle base head end and an inner wall of the accommodating room and is capable of being cut by the cutting blade segment. The needle base connecting rod is connected to the hard needle and is capable of pushing the needle plug of the needle set. The sealing unit assembled with the elastic unit is located in the connecting member. The sealing unit is located outside of the needle set mounting segment and covers the needle room. The sealing unit is tightly sleeved on the outer side of the needle base connecting rod. The elastic unit is sleeved on an outer side of the needle base connecting rod. Two ends of the elastic unit are respectively connected to the needle base head end of the needle base and the sealing unit. By this, in one single press applied on the needle output mechanism, the needle set protrudes from the base with the hard needle mounted in the soft needle. Additionally, the cutting blade segment of the connecting member cuts the connecting segment between the needle base and the needle output operating unit, and the elastic unit pushes the needle base to drive the hard needle to detach from the soft needle, so the soft needle is connected to the output adapter via the needle room and the connecting head, which facilitates ease in the operation of protruding the needle set.

Further, a diameter reducing segment is formed on an end, which is connected to the connecting base segment, of the connecting head of the connecting member, is formed with a reduced diameter and is capable of being cut off. A cutting knife segment is mounted on a side, which corresponds to the connecting head, of the needle output operating unit. The cutting knife segment is capable of being operated to cut off the diameter reducing segment of the connecting head of the connecting member. By this, when the abutting inclined surface of the needle returning operating unit of the needle output mechanism pushes the base board located on the back, which is away from the base, of the connecting member located in the base, the abutting inclined surface synchronously drives the connecting member and the soft needle mounted in the connecting member to retract to the base. Besides, the cutting knife segment of the needle output operating unit fixed on the base cuts the diameter reducing segment of the connecting head of the connecting member connected to the adapter mechanism, such that the needle output mechanism cannot be reused, thereby ensuring the safety in use.

The needle output mechanism may additionally have a safety cover and a safety bolt. The safety cover covers an outer side of the needle output operating assembly and is capable of engaging with the base. The safety bolt is mounted in the safety cover and the needle output operating unit of the needle output operating assembly. A pushing elastic unit is mounted between the safety cover and the needle output operating unit. Therefore, the needle output mechanism can be operated to protrude the needle only after the safety bolt is pulled out and the safety cover is detached, thereby ensuring safety in use. Besides, after the safety bolt is pulled out, the pushing elastic unit automatically pushes the safety cover away from the base, thereby enhancing operating convenience.

### In the drawings:

The following drawings are only intended to illustrate and explain the present invention and do not limit the scope of the present invention, wherein:
Fig. 1 is a perspective view of a preferred embodiment of an injector in accordance with the present invention;
Fig. 2 is a partially exploded perspective view of the preferred embodiment of the injector in Fig. 1;
Fig. 3 is a perspective view of a medicament container of the preferred embodiment of the injector in Figs. 1 and 2;
Fig. 4 is an operational perspective view of an injection module of the preferred embodiment of the injector in Figs. 1 and 2;
Fig. 5 is an exploded perspective view of a conversion mechanism of the injection module of the preferred embodiment of the injector in Figs. 1, 2, and 4;
Fig. 6 is an exploded perspective view from another viewing angle of the conversion mechanism of the injection module of the preferred embodiment of the injector in Figs. 1, 2, and 4;
Fig. 7 is a partial side view of the preferred embodiment of the injector in Figs. 1 and 2;
Fig. 8 is a sectional side view cut along line A-A in Fig. 7;
Fig. 9 is a sectional side view cut along line B-B in Fig. 7;
Fig. 10 is an exploded perspective view of a needle output mechanism of the injection module in Figs. 1, 2, and 4;
Fig. 11 is an exploded perspective view from another viewing angle of the needle output mechanism of the injection module in Figs. 1, 2, and 4;
Fig. 12 is a plan view of the needle output mechanism of the injection module in Figs. 1, 2, and 4;
Fig. 13 is a sectional side view cut along line C-C in Fig. 12;
Fig. 14 is a sectional side view cut along line D-D in Fig. 12;
Fig. 15 is an exploded perspective view of a driving mechanism of the preferred embodiment of the injector in Figs. 1 and 2;
Fig. 16 is an exploded perspective view from another viewing angle of the driving mechanism of the preferred embodiment of the injector in Figs. 1 and 2;
Fig. 17 is an operational view of the preferred embodiment of the injector in Figs. 1 and 2, showing the medicament container assembled to the injection module;
Fig. 18 is an operational view of the preferred embodiment of the injector in Figs. 1 and 2, showing a combination of the medicament container and the injection module assembled to the driving mechanism;
Fig. 19 is a sectional side view of the preferred embodiment of the injector in Figs. 1 and 2, showing the needle output mechanism of the injection module without a safety cover and a safety bolt;
Fig. 20 is an operational view of the needle output mechanism in Fig. 19, showing a needle output operating assembly being pressed and driving a needle set located in a connecting member to protrude out a base with a hard needle mounted in a soft needle;
Fig. 21 is an operational view of the needle output mechanism in Fig. 20, showing the needle output operating assembly fixed in the base, and a needle base detached from the needle output operating assembly to drive the hard needle to detach from the soft needle;
Fig. 22 is an operational view of the needle output mechanism in Fig. 19, showing a needle returning operating unit driving the soft needle to retract after injection;
Fig. 23 is an operational view (1) of the needle output mechanism in Fig. 21, showing the needle output mechanism cutting a diameter reducing segment of a connecting head segment of the connecting member during retracting the needle set; and
Fig. 24 is an operational view (2) of the needle output mechanism in Fig. 21, showing the needle output mechanism cutting the diameter reducing segment of the connecting head segment of the connecting member during retracting the needle set.

### Description of the Reference Numbers:

10: medicament container
11: container main body
12: container connecting port
121: female connecting port
122: buckling groove
13: back sticker
2: injection module
20: conversion mechanism
21: conversion connecting base
211: base body
212: valve base segment
213: input adapter
2131: male connecting port
2132: buckling hook
214: output adapter
215: connecting board
216: clamping segment
217: hook segment
218: back sticker
22: driving piston set
221: piston cylinder
2211: connecting end segment valve connecting end segment
2212: sliding groove
2213: buckling block
222: valve
2221: input one-way valve
2222: output one-way valve
223: piston rod
2231: sliding block
2232: L-shaped buckling groove
30: needle output mechanism
31: base
311: action room
312: base board
3121: assembling segment
313: penetration hole
314: through hole
315: action opening
316: fixing buckling hook
32: connecting member
321: connecting base segment
3211: connecting base annular wall
3212: needle set mounting segment
3213: needle room
3214: needle plug hole
3215: cutting blade segment
322: connecting head
3221: diameter reducing segment
33: needle set
331: soft needle
332: needle plug
333: hard needle
34: needle output operating assembly
341: needle output operating unit
3411: button segment
3412: cutting knife segment
342: needle base
3421: needle base head end
3422: needle base connecting rod
3423: connecting segment
343: sealing unit
3431: sealing gasket
3432: sleeving cover
344: elastic unit
35: needle returning operating unit
351: pressing segment
352: abutting inclined surface
353: extending segment
36: safety cover
37: safety bolt
38: pushing elastic unit
40: driving mechanism
41: case
411: connecting sleeve segment
4111: L-shaped buckling groove
412: case body segment
413: case body segment
414: fixing hook
42: driving unit
420: connecting end segment
421: engaging protrusion
422: restoring elastic unit
43: electrical driving unit
431: driving assembly
432: transmission assembly
4321: transmission rod
4322: gear assembly
4323: eccentric cam
44: controlling unit
441: controlling circuit board
442: display panel
443: controlling button
444: contact switch
45: power storage unit
46: electrically connecting member

In conjunction with the accompanying drawings and preferred embodiments of the present invention, the present invention is further elaborated on the technical means adopted for reaching the intended purpose of the invention.

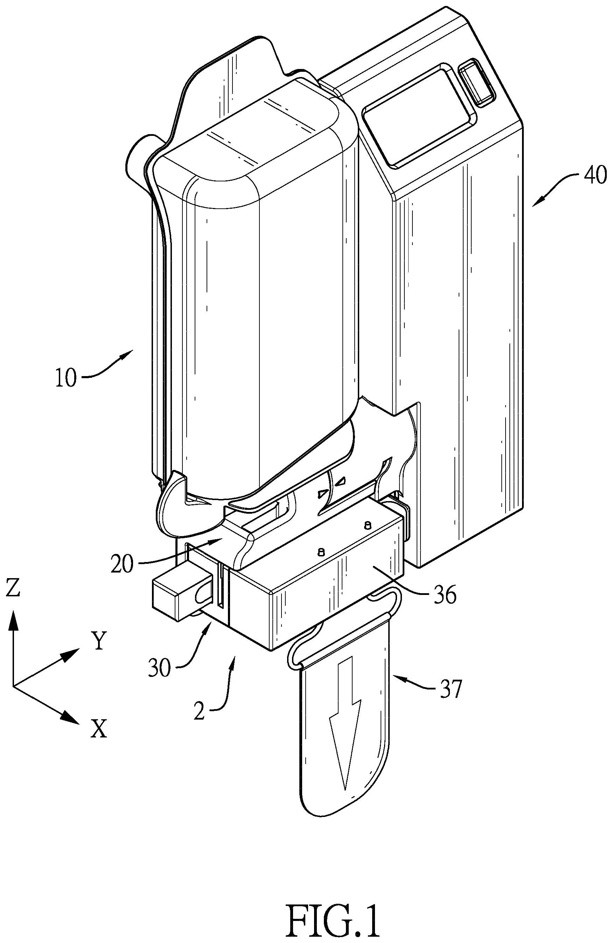

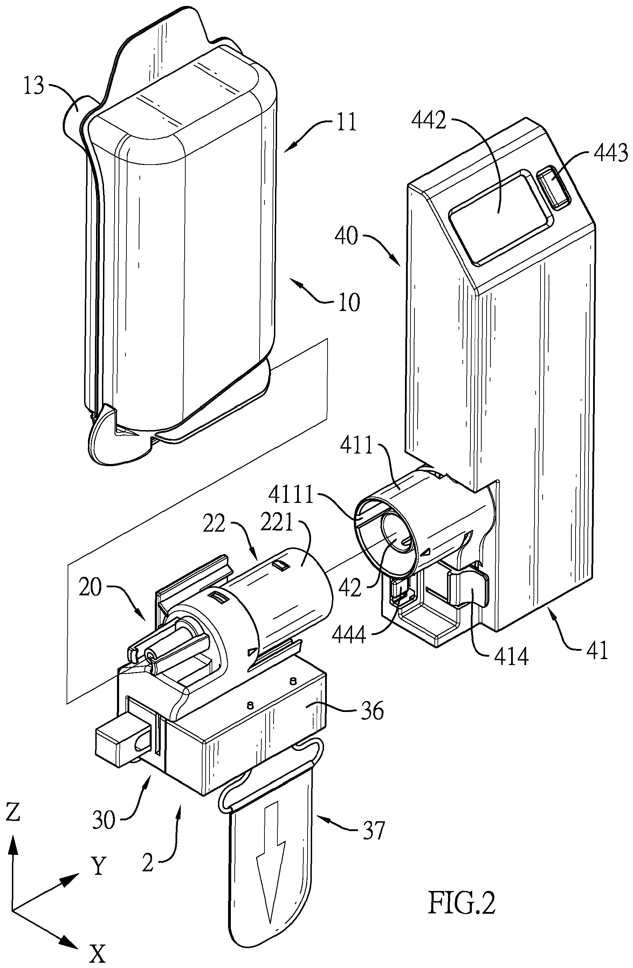

With reference to Figs. 1 and 2, an injector in accordance with the present invention is disclosed. The injector is located in a three-dimensional space having an X axis, a Y axis, and a Z axis. The injector has a front surface and a rear surface opposite to each other and is arranged along the X axis. The injector has a medicament container 10, an injection module 2, and a driving mechanism 40. These parts are detailed below.

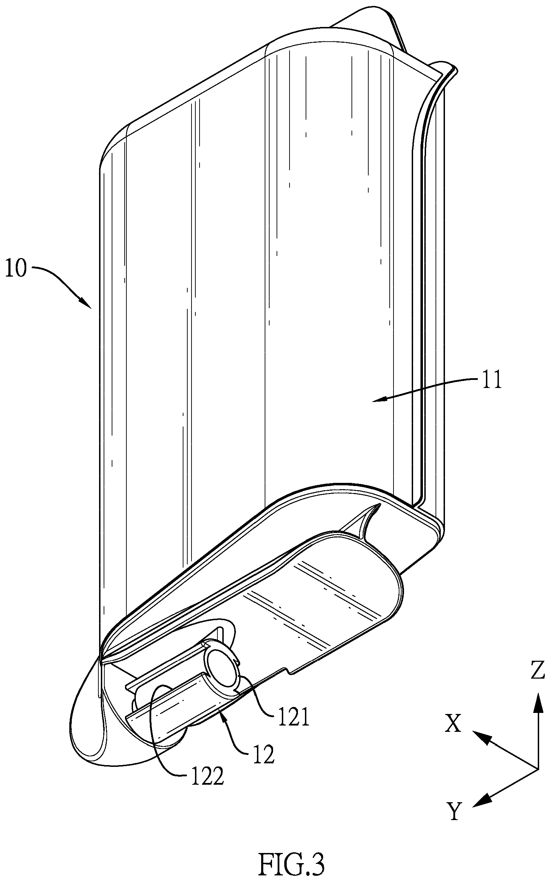

With reference to Figs. 1 to 3, the medicament container 10 is adapted to accommodate and seal a certain dose of medicament. The medicament container 10 has a container main body 11 and a container connecting port 12. The container main body 11 forms a space adapted to accommodate and seal the medicament. The container connecting port 12 is located on a side of the container main body 11 along a direction on the Z axis and is connected to an inner side of the container main body 11. The medicament filled in the container main body 11 can be output via the container connecting port 12. In this preferred embodiment, the medicament container 10 has a back sticker 13 mounted on the rear surface of the container main body 11 and having release paper, so the medicament container 10 can be stuck on items or a human body via the back sticker 13 after the release paper is removed. Or, the back sticker 13 can be replaced by a band, so the medicament container 10 can be mounted on items or a human body via the band. The medicament container 10 is capable of assembling with the injection module 2 via the container connecting port 12.

With reference to Figs. 1, 2, and 4, the injection module 2 has a conversion mechanism 20 and a needle output mechanism 30. The conversion mechanism 20 and the needle output mechanism 30 can be modular for facilitating assembling the injection module 2 with the medicament container 10 and the driving mechanism 40. A rear surface of the injection module 2 can be implemented with a back sticker having release paper, so that the injection module 2 can be stuck on items or a human body via the back sticker after the release paper is removed, or the back sticker can also be replace by band, and the injection module 2 is mounted on items or a human body by the band.

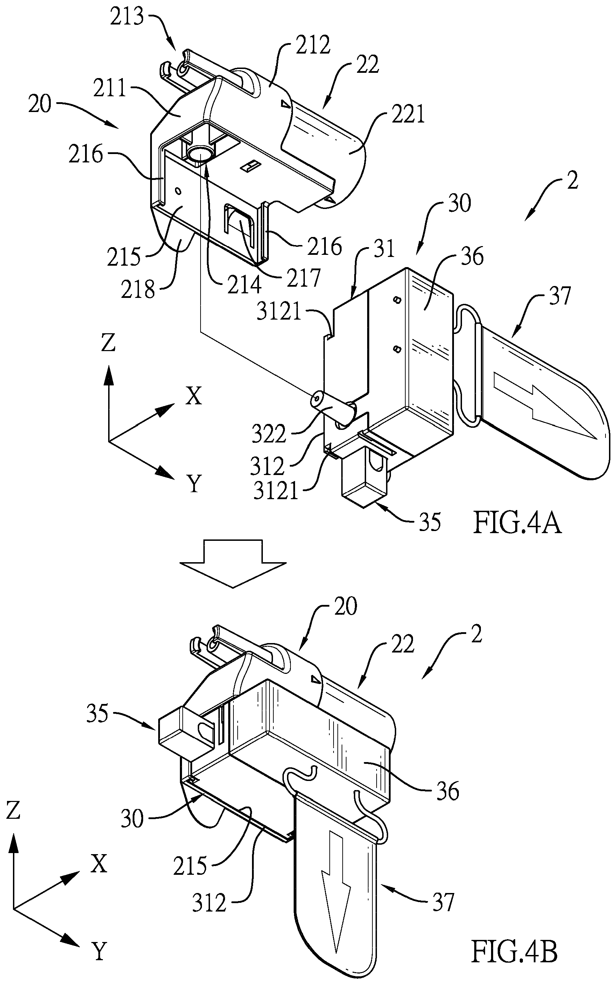

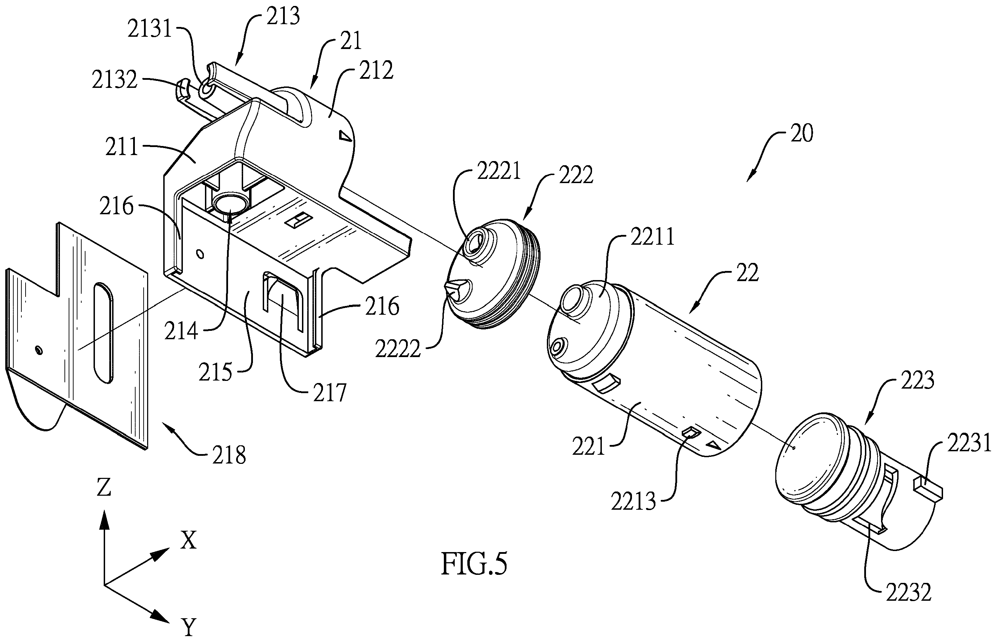

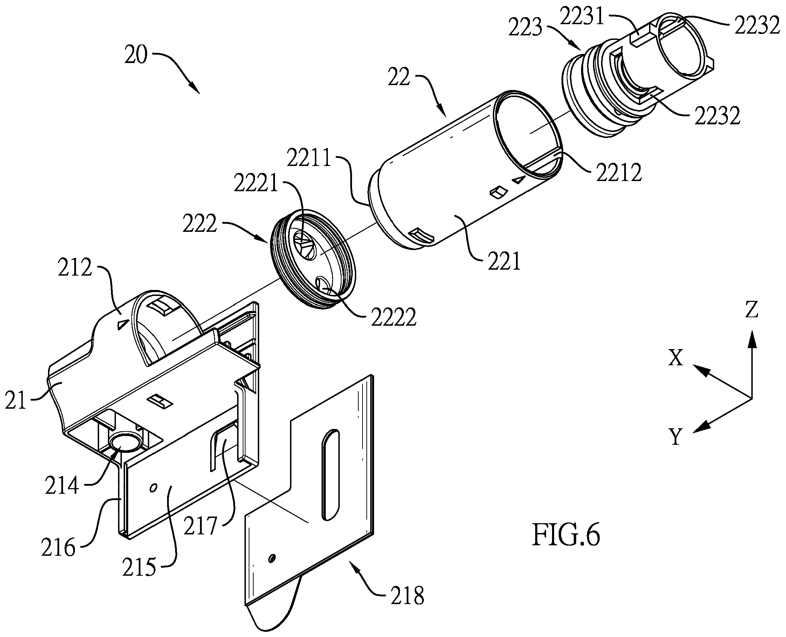

With reference to Figs. 4 to 6, the conversion mechanism 20 is connected to the medicament container 10 and the driving mechanism 40. The conversion mechanism 20 has a conversion connecting base 21 and a driving piston set 22. The conversion connecting base 21 has an input adapter 213 and an output adapter 214, and is connected to the medicament container 10 via the input adapter 213, and is connected to the needle output mechanism 30 via the output adapter 214. **In** this preferred embodiment, the conversion connecting base 21 has a base body 211 and a valve base segment 212. The valve base segment 212 is mounted on the base body 211. The valve base segment 212 has a sleeving opening toward outside. The sleeving opening is assembled with the driving piston set 22. The input adapter 213 is mounted on a side of the valve base segment 212 opposite to the sleeving opening. The output adapter 214 is mounted in the base body 211. The input adapter 213 and the output adapter 214 are connected to the sleeving opening of the valve base segment 212. The input adapter 213 is detachably connected to the container connecting port 12 of the medicament container 10. The output adapter 214 is connected to the needle output mechanism 30.

With reference to Figs. 4 to 6, in this preferred embodiment, the sleeving opening of the valve base segment 212 faces outwards along the Y axis. The input adapter 213 faces along the Y axis. The output adapter 214 faces along the Z axis. The driving piston set 22 is mounted in the sleeving opening of the valve base segment 212. The driving piston set 22 can be controlled to move and is capable of drawing and transporting the medicament from the medicament container 10 to the driving piston set 22, and then the medicament is output from the output adapter 214. The base body 211 has a connecting board 215 and is connected to the needle output mechanism 30 via the connecting board 215. The connecting board 215 has two clamping segments 216 located respectively on two opposite sides of the connecting board 215 on the Y axis. The connecting board 215 has a hook segment 217, so the connecting board 215 can engage with the needle output mechanism 30.

With reference to Figs. 4 to 6, in this preferred embodiment, the input adapter 213 of the conversion mechanism 20 has a male connecting port 2131 and at least one buckling hook 2132. The container connecting port 12 of the medicament container 10 has a female connecting port 121 and at least one buckling groove 122. The male connecting port 2131 of the input adapter 213 is matched and assembled with the female connecting port 121 of the container connecting port 12. The buckling hook 2132 of the input adapter 213 is matched and assembled with the buckling groove 122 of the container connecting port 12. The male connecting port 2131 and the female connecting port 121 can be implemented as standard male and female luer taper, which prevents leaking after assembled.

With reference to Figs. 4 to 6, in this preferred embodiment, a rear surface of the connecting board 215 of the base body 211 of the conversion connecting base 21 can be implemented with a back sticker 218 having release paper, so that the connecting board 215 can be stuck on items or a human body via the back sticker after the release paper is removed, or the back sticker can also be replaced with a band, and the connecting board 215 is mounted on items or a human body with the band.

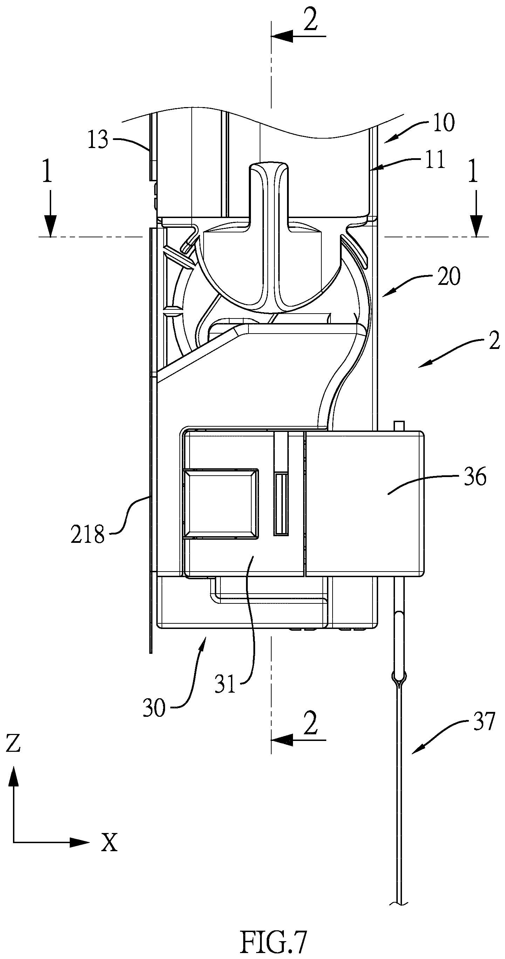

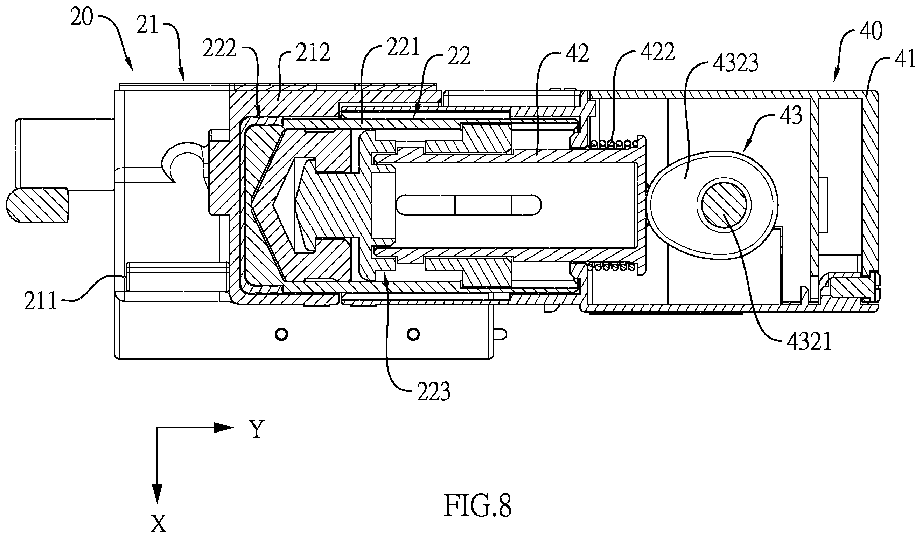

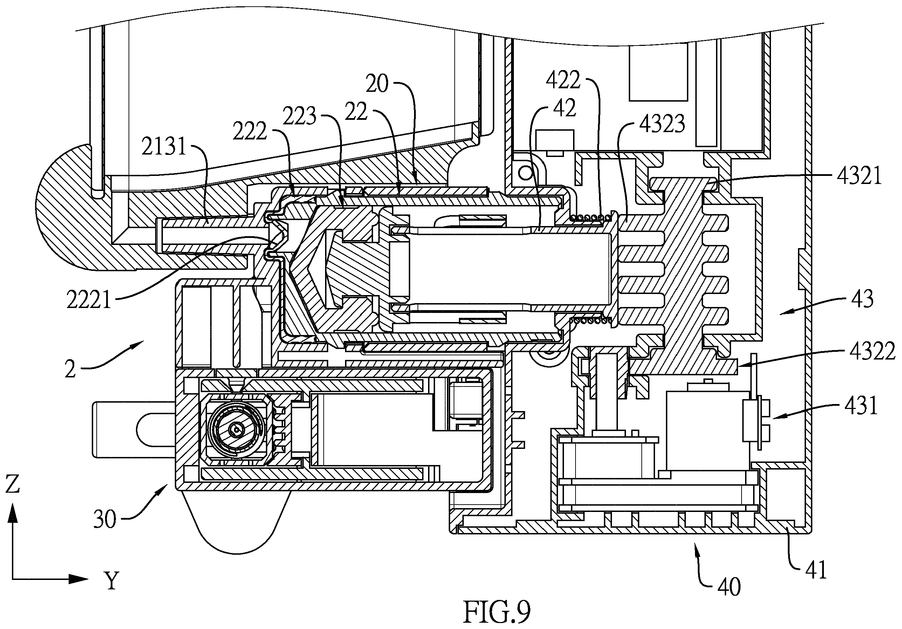

With reference to Figs. 2, 4 to 6 and 7 to 9, in this preferred embodiment, the driving piston set 22 has a piston cylinder 221, a valve 222, and a piston rod 223 mounted in the piston cylinder 221 and being capable of moving linearly along the Y axis. An end of the piston cylinder 221 has a valve connecting end segment 2211. The piston cylinder 221 has a piston room. The piston room extends along the Y axis to another end of the piston cylinder 221 and forms an opening. The piston cylinder 221 is assembled to the valve 222 via the valve connecting end segment 2211 and is mounted in the valve base segment 212. The valve 222 has an input one-way valve 2221 and an output one-way valve 2222. The input one-way valve 2221 is connected to the input adapter 213. The input one-way valve 2221 is only opened along a direction from the input adapter 213 to the valve base segment 212. The output one-way valve 2222 is connected to the output adapter 214. The output one-way valve 2222 is only opened along a direction from the valve base segment 212 to the output adapter 214. The input adapter 213 and the output adapter 214 of the conversion connecting base 21 are controllably connected to the piston room of the piston cylinder 221 respectively via the input one-way valve 2221 and the output one-way valve 2222 of the valve 222. The piston rod 223 is mounted in the piston cylinder 221 and is capable of moving linearly along the Y axis. The piston rod 223 is detachably connected to the driving mechanism 40, so the piston rod 223 can be driven by the driving mechanism 40.

With reference to Figs. 4 to 6, in this preferred embodiment, an inner cylinder wall of the piston cylinder 221 has at least one sliding groove 2212 extending along the Y axis. An outer annular surface of the piston rod 223 has at least one sliding block 2231. Each of the at least one sliding block 2231 corresponds in position to the sliding groove 2212 for guiding the piston rod 223 moving linearly along the Y axis in the piston cylinder 221. Besides, the piston rod 223 is a hollow rod and has at least one L-shaped buckling groove 2232, so the piston rod 223 and the driving mechanism 40 can be assembled by rotating to engage, and thus the piston rod 223 can be driven by the driving mechanism 40.

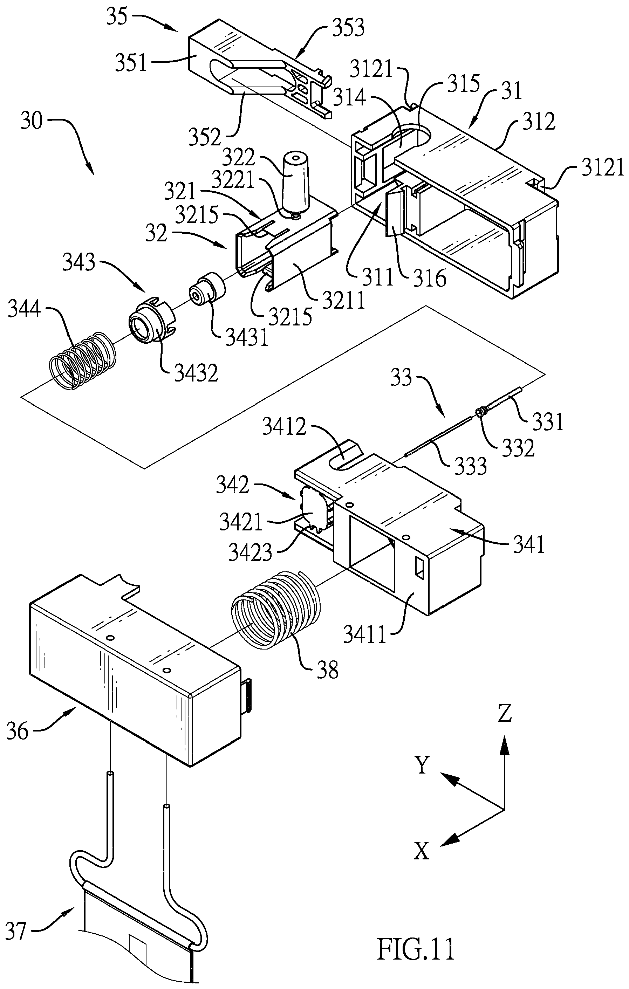

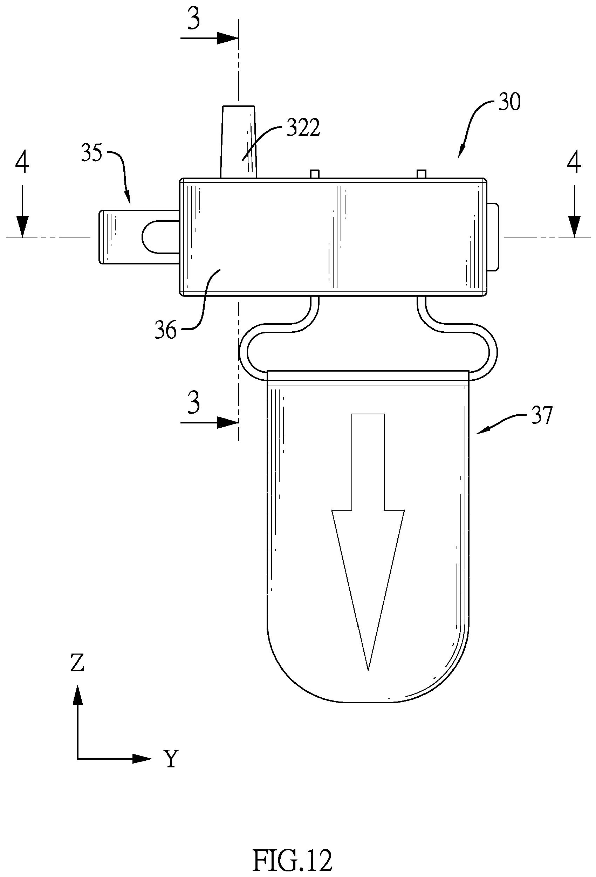

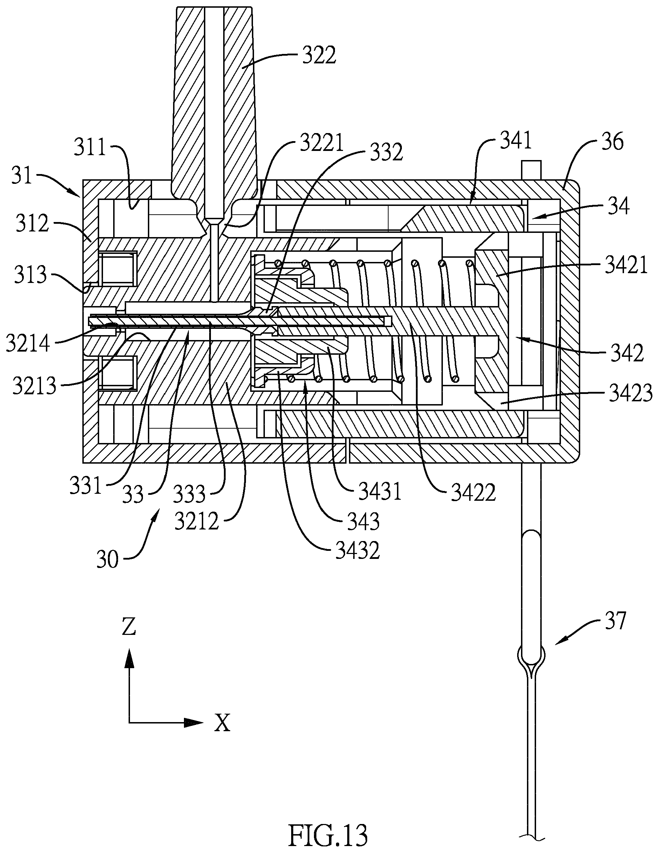

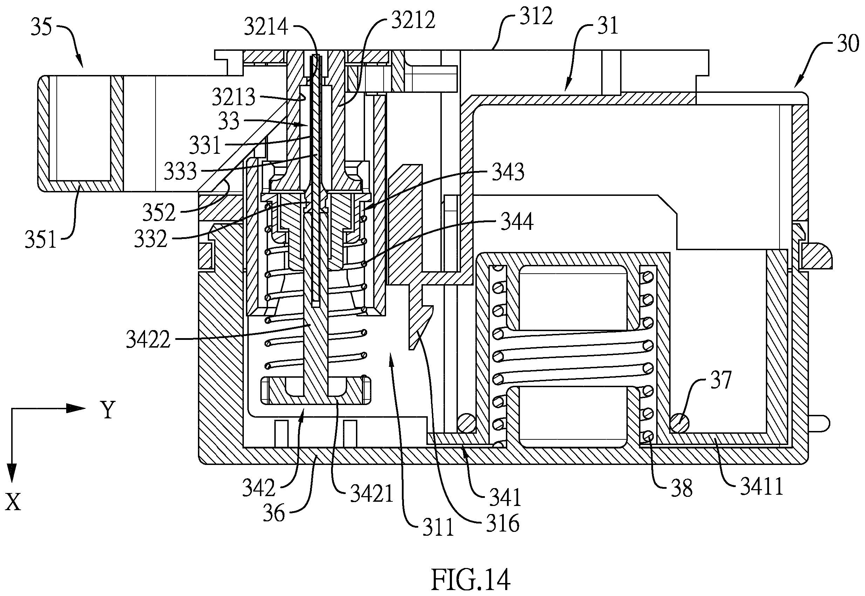

With reference to Figs. 2, 4 and 10 to 14, the needle output mechanism 30 is connected to the conversion mechanism 20 and is used for medicament injection. The needle output mechanism 30 has a base 31, a connecting unit 32, a needle set 33, a needle output operating assembly 34, and a needle returning operating unit 35.

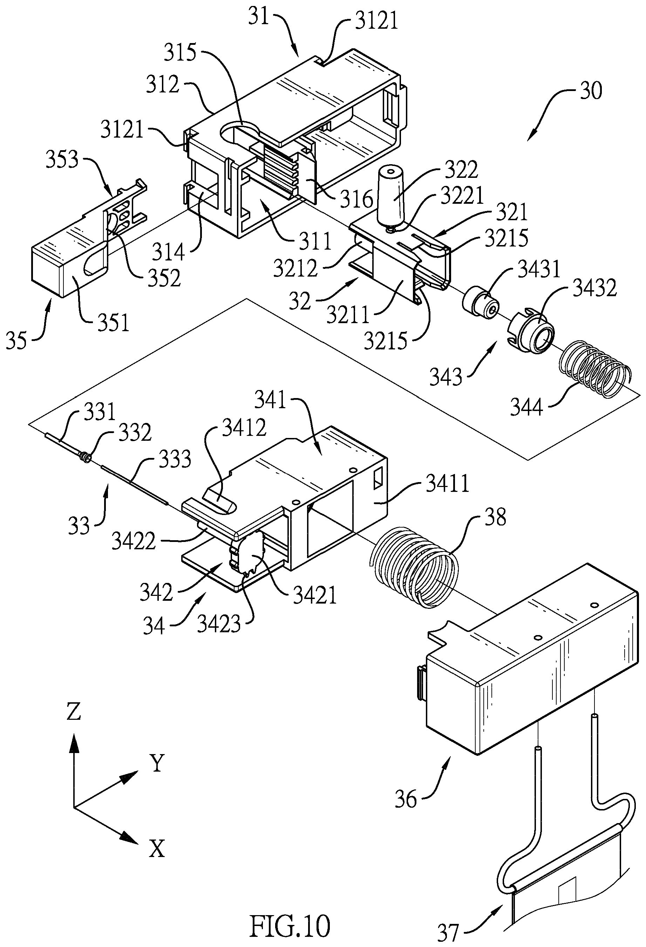

With reference to Figs. 10 to 14, the base 31 can be assembled with the base body 211 of the conversion mechanism 20. The base 31 has an action room 311 with an opening facing a front side along the X axis. The action room 311 has a base board 312 facing a rear side along the X axis. The base board 312 has a penetration hole 313 connected to the action room 311. The base 31 has a through hole 314 formed on a side of the action room 311 along the Y axis and connected to the action room 311. The base 31 has an action opening 315 formed on a side facing the conversion mechanism 20 and connected to the action room 311. The base board 312 has two assembling segments 3121 located on two opposite sides along the Y axis of the base board 312. The clamping segment 216 of the connecting board 215 of the conversion connecting base 21 of the conversion mechanism 20 is assembled with the assembling segment 3121. The hook segment 217 of the connecting board 215 is capable of engaging with the base board 312. The base 31 has a fixing buckling hook 316 mounted on an outer side of the action room 311.

With reference to Figs. 10 to 14, the connecting unit 32 is mounted in the action room 311 of the base 31 and is capable of moving along the X axis. The connecting unit 32 has a connecting base segment 321 and a connecting head 322. The connecting base segment 321 has a connecting base annular wall 3211 and a needle set mounting segment 3212 mounted in the connecting base annular wall 3211. The needle set mounting segment 3212 has a needle room 3213 inside. An end of the needle set mounting segment 3212 facing the base board 312 of the base 31 has a needle plug hole 3214. The needle plug hole 3214 corresponds to the penetration hole 313 of the base board 312. Meanwhile, the needle plug hole 3214 corresponds in position to the penetration hole 313. The connecting head 322 is mounted on the connecting base segment 321 and is connected to the needle room 3213 of the needle set mounting segment 3212. The connecting head 322 extends along the Z axis. In this preferred embodiment, an end of the connecting head 322 connected to the connecting base segment 321 forms a diameter reducing segment 3221 having a reducing diameter and adapted to be cut off. The connecting head 322 of the connecting unit 32 is mounted through the action opening 315 of the base 31 and is connected to the output adapter 214 of the conversion mechanism 20. The connecting base annular wall 3211 has at least one cutting blade segment 3215 located on a side of the connecting base annular wall 3211 away from the base board 312 of the base 31.

With reference to Figs. 10 to 14, the needle set 33 is mounted in the needle set mounting segment of the connecting unit 32, is capable of moving along the X axis, and can be operated to output and retract the needle. The needle set 33 has a soft needle 331, a needle plug 332, and a hard needle 333. The soft needle 331 is mounted in the needle room 3213 of the needle set mounting segment 3212 and is capable of moving along the X axis. An end of the soft needle 331 faces the needle plug hole 3214. Another end of the soft needle 331 is fixed to the needle plug 332. When the soft needle 331 is driven to protrude out of the base board 312 of the base 31, the needle plug 332 is stuck in the needle plug hole 3214. The hard needle 333 can be driven to be mounted in the needle plug 332 and the soft needle, and can protrude along with the soft needle 331. After the soft needle 331 is output, the hard needle 333 can be driven to detach from the soft needle 331, and then the soft needle 331 is connected to the conversion mechanism 20 via the needle room 3213 and the connecting head 322 for providing flow path for the medicament. The soft needle 331 can be retracted to the base 31 along with the connecting unit 32.

With reference to Figs. 10 to 14, the needle output operating assembly 34 is mounted in the base 31 and is capable of driving the needle set 33 to output the needle. The needle output operating assembly 34 has a needle output operating unit 341, a needle base 342, a sealing unit 343, and an elastic unit 344.

With reference to Figs. 10 to 14, the needle output operating unit 341 is mounted in the base 31 and is capable of moving along the X axis. The needle output operating unit 341 can be buckled and fixed by the fixing buckling hook 316 of the base 31. The needle output operating unit 341 has a button segment 3411 and an accommodating space located on a side of the button segment 3411. The accommodating space corresponds to the action room 311 of the base 31. Meanwhile, the accommodating space corresponds in position to the action room 311. A side of the needle output operating unit 341 corresponding to the connecting head 322 has a cutting knife segment 3412. The cutting knife segment 3412 can be operated to cut off the diameter reducing segment 3221 of the connecting head 322 of the connecting unit 32.

With reference to Figs. 10 to 14, the needle base 342 is mounted in the action room 311 of the needle output operating unit 341 and is connected to the needle output operating unit 341. After the needle is output, the needle base 342 is capable of being detached from the needle output operating unit 341. The needle base 342 has a needle base head end 3421 and a needle base connecting rod 3422. The needle base head end 3421 is connected to the needle output operating unit 341. The needle base connecting rod 3422 is formed on the needle base head end 3421. The needle base connecting rod 3422 is connected to the hard needle 333 and is capable of pushing the needle plug 332 of the needle set 33.

With reference to Figs. 10 to 14, in this preferred embodiment, the needle base 342 and the needle output operating unit 341 are formed integrally. A connecting segment 3423, which is adapted to be cut off, is formed between an edge of the needle base head end 3421 and the needle output operating unit 341. By the cutting blade segment 3215 of the connecting unit 32 mounted in the base 31 cutting off the connecting segment 3423, the needle base 342 and the needle output operating unit 341 are detached.

With reference to Figs. 10 to 14, the sealing unit 343 is assembled with the elastic unit 344 and is mounted in the connecting unit 32. The sealing unit 343 is mounted outside the needle set mounting segment 3212 and covers the needle room 3213. The sealing unit 343 sleeves and fits the needle base connecting rod 3422. The elastic unit 344 sleeves the needle base connecting rod 3422. Two ends of the elastic unit 344 are respectively connected to the needle base head end 3421 of the needle base 342 and the sealing unit 343. In this preferred embodiment, the sealing unit 343 has a sealing gasket 3431 and a sleeving cover 3432 sleeving the sealing gasket 3431. The elastic unit 344 abuts the sleeving cover 3432. The sealing gasket 3431 sleeves and fits the needle base connecting rod 3422.

With reference to Figs. 10 to 14, after the needle output operating unit 341 is pressed toward the base 31, the hard needle 333 of the needle set 33 is mounted in the soft needle 331, so the needle output operating unit 341 drives the needle base 342 to push the needle set to protrude out of the base board 312 of the base 31 and press the connecting unit 32 via the elastic unit 344, and thus makes the connecting unit 32 abut the base board 312 located on the rear surface of the base 31, such that the soft needle 331 can pierce into a human body with the hard needle 333 mounted inside. After the needle plug 332 is stuck in the needle plug hole 3214 of the needle set mounting segment 3212, the needle output operating unit 341 is buckled and fixed by the fixing buckling hook 316 in the base 31. The connecting segment 3423 on the edge of the needle base 342 is cut off by the cutting blade segment 3215 of the connecting unit 32. The needle base 342 is pushed out by the elastic unit 344, and then the hard needle 333 is driven to be pulled out of the soft needle 331 by the needle base connecting rod 3422 of the needle base 342, such that the soft needle 331 is connected to the conversion mechanism 20 via the needle room 3213 of the connecting unit and the connecting head 322.

With reference to Figs. 10 to 14, the needle returning operating unit 35 extends from the outer side of the base 31 into the action room 311 via a through hole. The needle returning operating unit 35 has a pressing segment 351, an abutting inclined surface 352 and an extending segment 353. The pressing segment 351 is located outside the base 31. The abutting inclined surface 352 is located on the side where the pressing segment 351 extends into the action room 311. The abutting inclined surface 352 abuts the connecting base annular wall 3211 of the connecting unit 32. The extending segment 353 is located on the side where the pressing segment 351 extends into the action room 311 and bypasses the needle set mounting segment 3212 of the connecting unit 32. A position limiting hook is formed on an end of the extending segment 353. The base 31 limits the position of the position limiting hook such that the needle returning operating unit 35 is mounted in the base 31 and is restricted to move within a limited distance without detaching the base 31. When the pressing segment 351 of the needle returning operating unit 35 is pressed, the abutting inclined surface 352 pushes the connecting unit 32 in the base 31 away from the base board 312 on the back of the base 31, thereby retracting the connecting unit 32 and the soft needle 331 of the needle set 33 mounted in the connecting unit 32 back into the base 31.

With reference to Figs. 10, 11 and 14, the needle output mechanism 30 may be further implemented with a safety cover 36 and a safety bolt 37 to ensure the safety of the needle output mechanism 30. The safety cover 36 covers the needle output operating assembly 34 and is capable of engaging the base 31 for fixing. The safety bolt 37 is mounted in the safety cover 36 and the needle output operating unit 341 of the needle output operating assembly 34. Thus, the needle output mechanism 30 is allowed to output needles only after the safety bolt 37 and the safety cover 36 are removed for ensuring the safety of use. In addition, a pushing elastic unit 38 is mounted between the safety cover 36 and the needle output operating unit 341, so after the safety bolt 37 is pulled out, the safety cover 36 can be automatically pushed away from the base 31 by the pushing elastic unit 38, thereby facilitating operation.

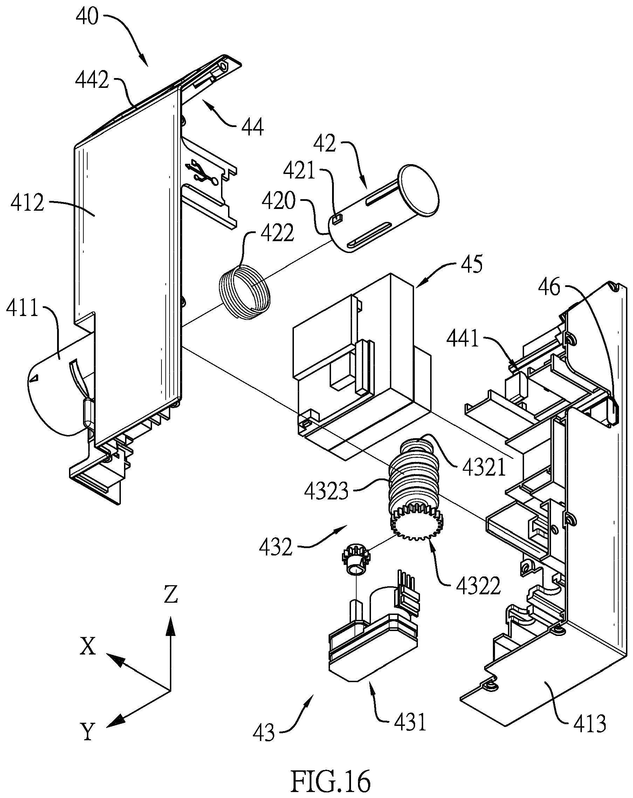

With reference to Figs. 1, 2, 15, and 16, the driving mechanism 40 is assembled with the conversion mechanism 20 of the injection module, and is capable of driving the movement of the piston rod 223 in the conversion mechanism 20. The driving mechanism 40 can be a manual driving mechanism or an electric driving mechanism. Preferably, the driving mechanism 40 is detachably connected to the conversion mechanism 20 of the injection module, so that the driving mechanism 40 can be reused. The driving mechanism 40 has a case 41 and a driving unit 42 mounted in the case 41. The case 41 is detachably connected to the conversion connecting base 21 of the conversion mechanism 20. The driving unit 42 is detachably connected to the piston rod 223. The driving unit 42 is capable of manually or electrically driving the piston rod 223 to move.

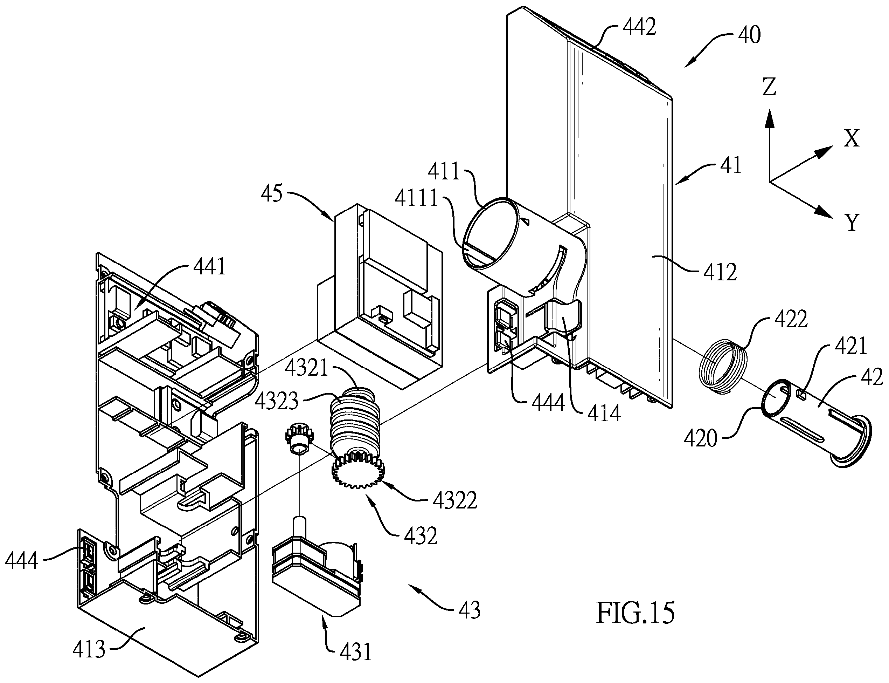

With reference to the preferred embodiment shown in Figs. 15 to 16, the case 41 is a hollow body with a space inside. A side of the case 41 has a connecting sleeve segment 411. The connecting sleeve segment 411 has a shaft hole extending from an outer side into the case 41. The connecting sleeve segment 411 is detachably sleeved on the piston cylinder 221 of the conversion mechanism 20.

With reference to Figs. 15 to 16, in this preferred embodiment, the case 41 is composed of multiple case body segments 412, 413 in a screw locking, clipping or bonding manner. The connecting sleeve segment 411 is rotatably assembled with the piston cylinder 221. An outer peripheral surface of the piston cylinder 221 is implemented with a buckling block 2213, and the connecting sleeve segment 411 is implemented with an L-shaped buckling groove 4111. By the buckling block 2213 and the L-shaped buckling groove 4111, the connecting sleeve segment 411 and the piston cylinder 221 are rotatably assembled with each other.

With reference to Figs. 15 to 16, 8 and 9, the driving unit 42 is mounted in the connecting sleeve segment 411 and is capable of moving straightly. An end of the driving unit 42 facing the connecting sleeve segment 411 forms a connecting end segment 420. The connecting end segment 420 is detachably connected to the piston rod 223. In this preferred embodiment, an outer peripheral surface of the connecting end segment 420 of the driving unit 42 has at least one engaging protrusion 421. The driving unit 42 can be mounted in the piston rod 223 and matches the L-shaped buckling of the engaging protrusion 421 via the engaging protrusion 421, so that the piston rod 223 and the driving mechanism 40 are rotatably assembled with each other.

With reference to Figs. 15 to 16, in this preferred embodiment, the driving mechanism 40 is an electric driving mechanism, but not limited thereto. When the driving mechanism 40 is an electric driving mechanism, the driving mechanism 40 has the case 41 and the driving unit 42 mounted in the case 41, and further has an electrical driving unit 43 and a controlling unit 44, or a power storage unit 45.

With reference to Figs. 15 to 16, the electrical driving unit 43 is mounted in the case 41 and can be connected to the driving unit 42 in the connecting sleeve segment 411 of the case 41. The electrical driving unit 43 has a driving assembly 431 and a transmission assembly 432. The driving assembly 431 can be an electric driving mechanism including a motor and a pump or other electric rotating driving mechanism. The transmission assembly 432 connects the driving assembly 431 and the driving unit 42, so that the driving assembly 431 can drive the driving unit 42 via the transmission assembly 432. In this preferred embodiment, the transmission assembly 432 has a transmission rod 4321 and a gear assembly 4322. The transmission rod 4321 is pivotally mounted in the case 41. The transmission rod 4321 has at least one eccentric cam 4323. The gear assembly 4322 has multiple gears engaging with each other. The gear assembly 4322 is connected between the driving assembly 431 and the transmission rod 4321. The eccentric cam 4323 of the transmission rod 4321 is connected to another end of the driving unit 42 located in the case 41. A retreating elastic unit 422 is mounted between the driving unit 42 and the case 41 for driving the driving unit 42 and the connected piston rod 223 to reciprocate linearly. In this preferred embodiment, the restoring elastic unit 422 is a compressed elastic unit. The restoring elastic unit 422 is sleeved on the driving unit 42. The end of the driving unit 42 located in the case 41 has an abutting flange. Two ends of the retreating elastic unit 422 respectively abut the case 41 and the abutting flange of the driving unit 42.

With reference to Figs. 15 to 16, the controlling unit 44 is mounted in the case 41, and the controlling unit 44 has a controlling circuit board 441 and a display panel 442. The display panel 442 is electrically connected to the controlling circuit board 441. The display panel 442 is exposed from a front surface of the case 41. The display panel 442 can be a conventional display panel having a display function. The controlling unit 44 also has at least one controlling button 443. The controlling button 443 is located on the front surface of the case 41 and is located on a side of the display panel 442. The controlling button 443 is electrically connected to the controlling circuit board 441, so that the user can switch, set and operate via the controlling button 443. Alternatively, the display panel 442 can also be a touch-sensitive display panel. The touch-sensitive display panel not only displays the working status, but also has user operation definition, allowing users to set up and operate on the display panel.

With reference to Figs. 15 to 16, in addition, the controlling unit 44 also has at least one contact switch 444. The contact switch 444 is mounted on a side of the case 41 adjacent to the needle output mechanism 30, and is electrically connected the controlling circuit board 441. When the driving mechanism 40 is connected to the conversion mechanism 20 of the injection module 2, the needle output mechanism 30 of the injection module 2 can touch the contact switch 444 so that the controlling unit 44 is switched from off to on state. The driving mechanism 40 can also be implemented with a fixing hook 414 on the case 41 at a position adjacent to the contact switch 444. The base 31 of the needle output mechanism 30 can be fixed on the case 41 by the fixing hook 414, so that the driving mechanism 40 and the injection module 2 are firmly assembled.

With reference to Figs. 15 to 16, the controlling unit 44 can be connected to an external power supply or the power storage unit 45, and the external power supply or the power storage unit 45 can provide the electric energy required for the driving mechanism 40 to operate. In the preferred embodiment shown in Fig. 15 to Fig. 16, the driving mechanism 40 also has a power storage unit 45 mounted in the case 41 and electrically connected to the controlling circuit of the controlling circuit board 441. The power storage unit 45 can be a wireless rechargeable power storage unit, a wired rechargeable power storage unit or any other non-rechargeable power storage unit (for example: battery). In the preferred embodiment shown in Figs. 15 to 16, the power storage unit 45 is a rechargeable power storage unit, such as a lithium battery. The power storage unit 45 is electrically connected to an electrically connecting unit 46. The electrically connecting unit 46 is exposed from the case 41, and can be connected to an external power source through a connection line for recharging. The electrically connecting unit 46 can be a universal serial bus (Universal Serial Bus, USB) type electrically connecting unit or other types of electrically connecting unit.

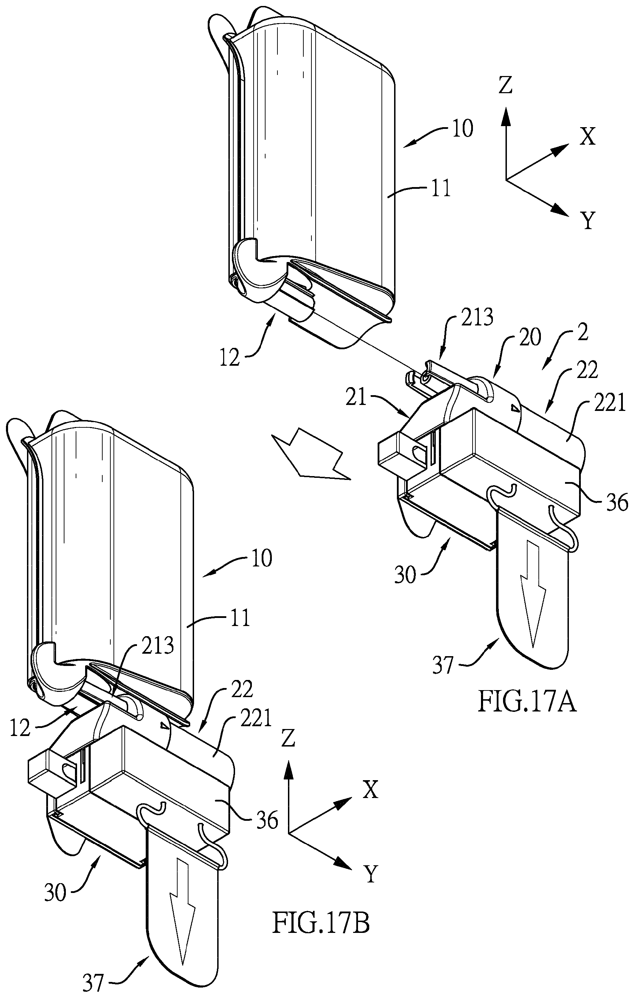

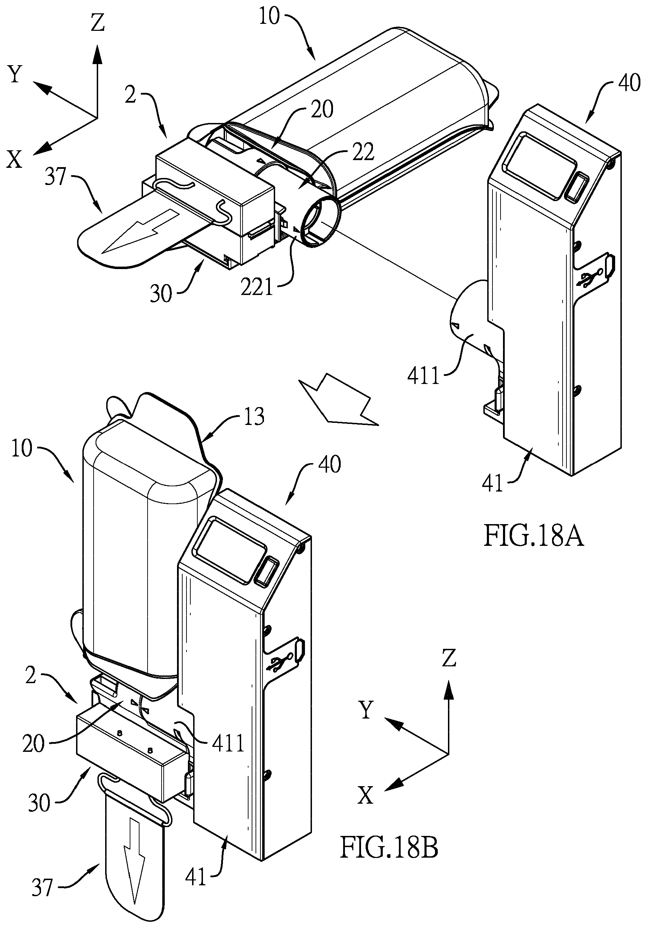

Regarding the use of the injector of the present invention, with reference to Figs. 17 and 18. The conversion mechanism 20 and the needle output mechanism 30 have been pre-assembled into an injection module 2 upon shipping out. Before the user performs subcutaneous injection, the medicament container 10 with the injection medicament and the driving mechanism 40 are sequentially assembled to the conversion mechanism 20 of the injection module 2. Herein, the medicament container 10 is assembled with the input adapter 213 of the conversion mechanism 20 of the injection module 2 via the container connecting port 12, and then the driving mechanism 40 is connected to the conversion mechanism 20. Wherein, the connecting sleeve segment 411 of the driving mechanism 40 is connected to the piston cylinder 221 of the conversion mechanism 20 by rotational engagement. At the same time, the driving unit is connected to the piston rod 223. After the injection module 2 rotates to a predetermined position, the needle output mechanism 30 contacts the contact switch 444 of the driving mechanism 40, such that the controlling unit 44 of the driving mechanism 40 is switched from the off state to the on state.

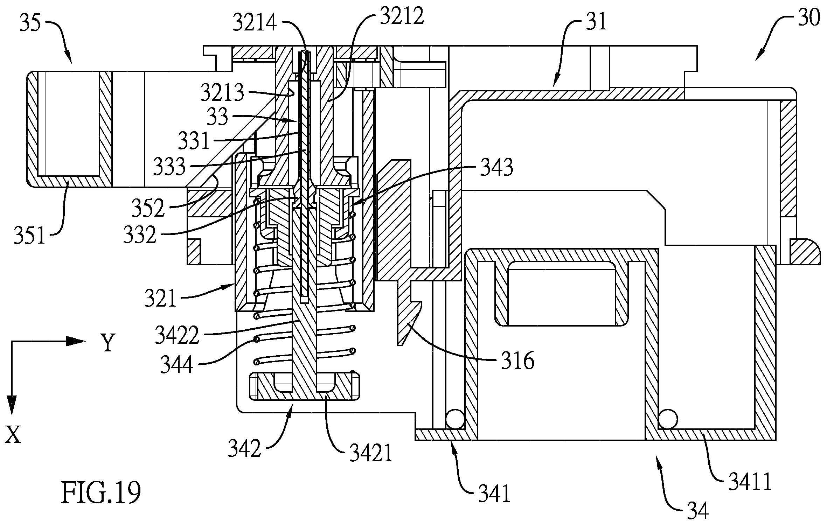

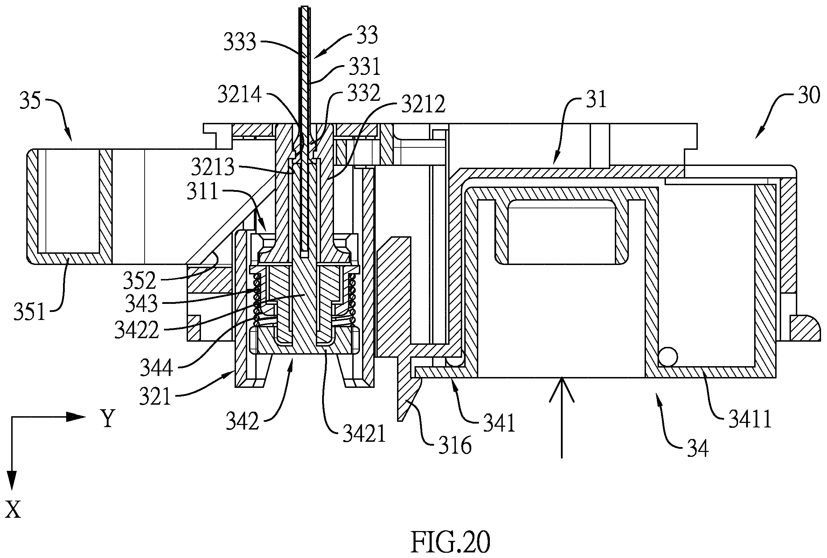

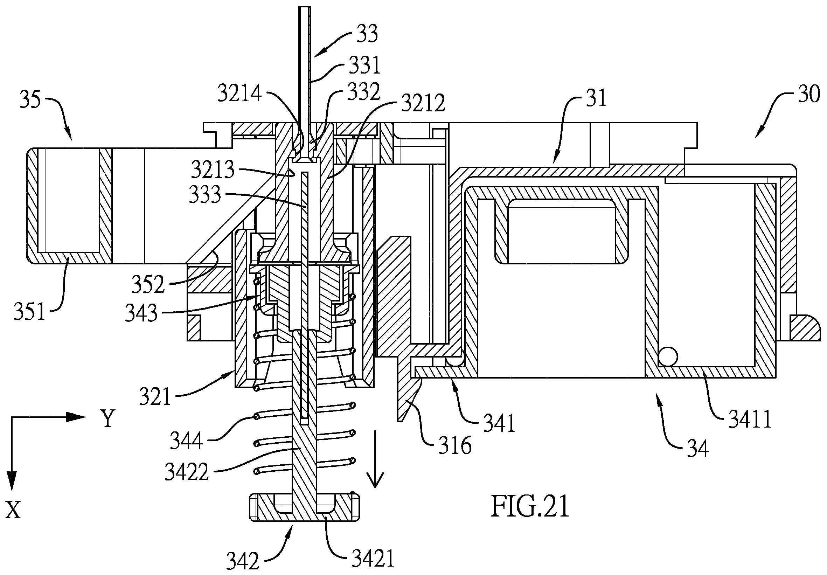

With reference to Figs. 18 to 21, before injection, the medicament container 10 and the injection module 2 are fixed on the intended injection site of the human body by the back sticker 13 or strap, and then remove the safety bolt 37 and safety cover 36 of the needle output mechanism 30 of the injection module 2. Next, press the needle output operating unit 341 of the needle output mechanism 30 to fix the needle output operating unit 341 on the base 31 and to push the needle set 33 via the needle base 342 and make the needle set 33 protrude with the hard needle 333 mounted in the soft needle 331 and pierce directly into the human body. On the other hand, the connecting segment 3423 between the needle base 342 and the needle output operating unit 341 is cut off by the cutting blade segment 3215 of the connecting unit 32. The elastic unit 344 pushes the needle base 342 and pulls out the hard needle 333 from the soft needle 331. The soft needle 331 is left in the human body and is connected to the output adapter 214 of the conversion mechanism 20 via the needle room 3213 and the connecting head 322. Later, the user is allowed to operate the driving mechanism 40 to drive the driving piston set 22 of the conversion mechanism 20 for drawing the medicament from the medicament container 10, conveyed to the needle output mechanism 30, and injected into the human body via the soft needle 331. After the injection is completed, stop the driving mechanism 40 from driving the driving piston set 22 of the conversion mechanism 20. In this way, with the injector fixed on the human body and the soft needle 331 staying in the state of piercing the human body, the user can regularly perform multiple injections through the driving mechanism 40.

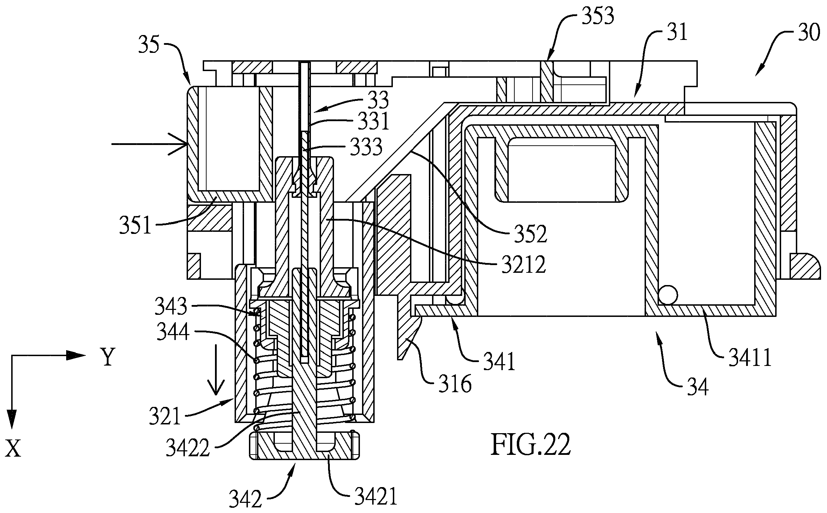

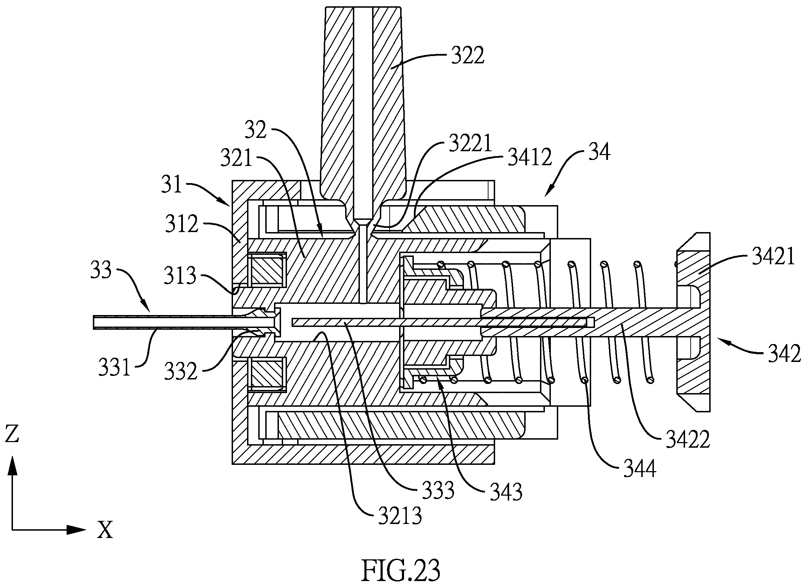

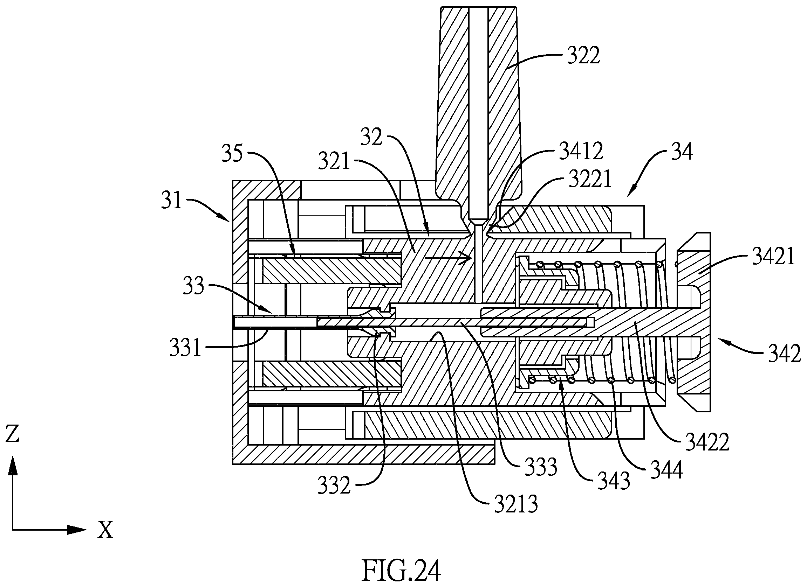

After the medicament in the medicament container 10 is used up, as shown in Fig. 22, the user can press the needle return operating unit 35 of the needle output mechanism 30 and use the abutting inclined surface 352 of the needle return operating unit 35 to push the connecting unit 32 in the base 31 away from the base board 312 on the back of the base 31, and at the same time drives the soft needle 331 of the needle set 33 in the connecting unit 32 to be pulled out from the human body and retracted into the base 31 together. With reference to Figs. 23 and 24, at this time, while the abutting inclined surface 352 of the needle returning operating unit 35 is pushing the connecting unit 32, the diameter reducing segment 3221 of the connecting head 322 connected to the connecting unit 32 is cut off by the cutting knife segment 3412 of the needle output operating unit 341 fixed on the base 31, such that the injection module 2 cannot be reused to ensure the safety of the injector. After the injector is used, the driving mechanism 40 can be removed from the injection module 2 for another use.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. An injector, comprising:
a medicament container (10) having a container main body (11) accommodating medicament and a container connecting port (12) mounted on a side of the container main body (11);
an injection module (2) having
a conversion mechanism (20) connected to the medicament container (10); the conversion mechanism (20) having a conversion connecting base (21) and a driving piston set (22) mounted in the conversion connecting base (21); the conversion connecting base (21) having an input adapter (213) and an output adapter (214); the conversion connecting base (21) connected to the container connecting port (12) of the medicament container (10) via the input adapter (213); and
a needle output mechanism (30) connected to the conversion mechanism (20); the needle output mechanism (30) having a needle set (33); the needle set (33) adapted to be connected to the output adapter (214); and
a driving mechanism (40) detachably connected to the conversion mechanism (20) of the injection module (2); the driving mechanism (40) having a case (41) and a driving unit (42) mounted in the case (41) and adapted to be operated to move; the case (41) detachably connected to the conversion connecting base (21) of the conversion mechanism (20); the driving unit (42) connected to the driving piston set (22) and being capable of driving the driving piston set (22) to draw and transport medicament from the medicament container (10) to the needle output mechanism (30), and then injecting the medicament with the needle set (33) of the needle output mechanism (30);
the injector **characterized in that** the needle output mechanism (30) has:
a base (31) connected to the conversion connecting base (21);
a connecting member (32) moveably mounted in the base (31) and connected to the output adapter (214) of the conversion mechanism (20), wherein the needle set (33) is moveably mounted in the connecting member (32) and has a soft needle (331), a needle plug (332) mounted on an end of the soft needle (331), and a hard needle (333) capable of being mounted in the soft needle (331);
a needle output operating assembly (34) moveably mounted in the base (31) and connected to the needle set (33); the needle output operating assembly (34) adapted to be operated to drive the needle set (33) located in the connecting member (32) to protrude out of the base (31) with the hard needle (333) mounted in the soft needle (331), and then drive the hard needle (333) to detach from the soft needle (331) to make the soft needle (331) connect to the output adapter (214) of the conversion mechanism (20) via the connecting member (32); and
a needle returning operating unit (35) moveably mounted in the base (31); the needle returning operating unit (35) adapted to be operated to drive the connecting member (32) and the soft needle (331) mounted in the connecting member (32) to retract back to the base (31).

2. The injector as claimed in claim 1, and **characterized in that** the base (31) of the needle output mechanism (30) has an action room (311); a back surface of the base (31) has a base (31) board and a penetration hole (313) formed on the base (31) board and connected to the action room (311); a side, which faces toward the conversion mechanism (20), of the base (31) has an action opening (315) connected to the action room (311); and a fixing buckling hook (2132) is mounted on the base (31) and located outside the action room (311);
the connecting member (32) is moveably mounted in the action room (311) of the base (31); the connecting member (32) has a connecting base (31) segment and a connecting head (322); the connecting base (31) segment has a base (31) annular wall and a needle set (33) mounting segment (3212) mounted in the base (31) annular wall; the needle set (33) mounting segment (3212) has a needle room (3213) formed in the needle set (33) mounting segment (3212) and a needle plug (332) hole (3214) formed on an end, which faces toward the base (31) board, of the needle set (33) mounting segment (3212) and connected to the needle room (3213); the needle plug (332) hole (3214) corresponds to the penetration hole (313) of the base (31) board; the connecting head (322) is mounted on the connecting base (31) and connected to the needle room (3213) in the needle set (33) mounting segment (3212); the connecting head (322) is mounted through the action opening (315) of the base (31) and is connected to the output adapter (214) of the conversion mechanism (20); the base (31) annular wall has at least one cutting blade segment (3215);
the soft needle (331) of the needle set (33) is moveably mounted in the needle room (3213) of the needle set (33) mounting segment (3212); the needle plug (332) that is mounted on the soft needle (331) is capable of being fixed in the needle plug (332) hole (3214); and
the needle output operating assembly (34) has a needle output operating unit (341), a needle base (31), a sealing unit (343), and an elastic unit (344); the needle output operating unit (341) is moveably mounted in the base (31), and the needle output operating unit (341) is capable of being fixed and buckled by the fixing buckling hook (2132) of the base (31); the needle output operating unit (341) having a button segment (3411) and an accommodating room located on a side of the button segment (3411); the accommodating room corresponds to the action room (311); the needle base (31) is located in the accommodating room of the needle output operating unit (341) and is connected to the needle output operating unit (341); the needle base (31) has a needle base (31) head end and a needle base (31) connecting rod formed on the needle base (31) head end; a connecting segment (3423) is mounted between a periphery of the needle base (31) head end and an inner wall of the accommodating room of the needle output operating unit (341) and is capable of being cut by the cutting blade segment (3215); the needle base (31) connecting rod is connected to the hard needle (333) and is capable of pushing the needle plug (332) of the needle set (33); the sealing unit (343) assembled with the elastic unit (344) is located in the connecting member (32); the sealing unit (343) is located outside of the needle set (33) mounting segment (3212) and covers the needle room (3213); the sealing unit (343) is tightly sleeved on an outer side of the needle base (31) connecting rod; the elastic unit (344) is sleeved on the outer side of the needle base (31) connecting rod; two ends of the elastic unit (344) are respectively connected to the needle base (31) head end of the needle base (31) and the sealing unit (343).

3. The injector as claimed in claim 2, and **characterized in that** a diameter reducing segment (3221) is formed on an end, which is connected to the connecting base (31) segment, of the connecting head (322) of the connecting member (32), is formed with a reduced diameter and is capable of being cut off; a cutting knife segment (3412) is mounted on a side, which corresponds to the connecting head (322), of the needle output operating unit (341); the cutting knife segment (3412) is capable of being operated to cut off the diameter reducing segment (3221) of the connecting head (322) of the connecting member (32).

4. The injector as claimed in claim 2, and **characterized in that** a through hole (314) is formed on a side, which is adjacent to the action room (311), of the base (31) and is connected to the action room (311); the needle returning operating unit (35) extends into the action room (311) from an outer side of the base (31) via the through hole (314); the needle returning operating unit (35) has a pressing segment (351), an abutting inclined surface (352) and an extending segment (353); the pressing segment (351) extends to the outer side of the base (31); the abutting inclined surface (352) is located on a side, which extends into the action room (311), of the pressing segment (351); the abutting inclined surface (352) abuts the base (31) annular wall of the connecting member (32); the extending segment (353) is located on the side, which extends into the action room (311), of the pressing segment (351) and bypassing the needle set (33) mounting segment (3212) of the connecting member (32);
a diameter reducing segment (3221) is formed on an end, which is connected to the connecting base (31) segment, of the connecting head (322) of the connecting member (32), is formed with a reduced diameter and is capable of being cut off; a cutting knife segment (3412) is mounted on a side, which corresponds to the connecting head (322), of the needle output operating unit (341); the cutting knife segment (3412) is capable of being operated to cut off the diameter reducing segment (3221) of the connecting head (322) of the connecting member (32).

5. The injector as claimed in any one of claims 1 to 4, and **characterized in that** the needle output mechanism (30) further has a safety cover (36) and a safety bolt (37); the safety cover (36) covers an outer side of the needle output operating assembly (34) and is capable of engaging with the base (31); the safety bolt (37) is mounted in the safety cover (36) and the needle output operating unit (341) of the needle output operating assembly (34); a pushing elastic unit (344) is mounted between the safety cover (36) and the needle output operating unit (341).

6. The injector as claimed in any one of claims 1 to 4, and **characterized in that** the conversion connecting base (21) has a base (31) body (211) and a valve (222) base (31) segment (212) mounted on the base (31) body (211); the valve (222) base (31) segment (212) has a sleeving opening; the input adapter (213) is mounted on the valve (222) base (31) segment (212) and is located on a side opposite to the sleeving opening; the output adapter (214) is mounted in the base (31) body (211); the input adapter (213) and the output adapter (214) are connected to the sleeving opening; the driving piston set (22) has a piston cylinder (221) having a piston room, a valve (222), and a piston rod (223) being capable of moving linearly in the piston cylinder (221); the piston cylinder (221) has a valve (222) connecting end segment (420) located on an end of the piston cylinder (221); the valve (222) connecting end segment (420) is assembled with the valve (222) and is mounted in the valve (222) base (31) segment (212); the valve (222) has an input one-way valve (222) connected to the input adapter (213) and an output one-way valve (222) connected to the output adapter (214); the piston rod (223) is mounted in the piston cylinder (221) and is capable of moving linearly; the piston rod (223) is detachably connected to the driving unit (42) of the driving mechanism (40).

7. The injector as claimed in of claim 6, and **characterized in that** the case (41) has a connecting sleeve segment (411) located on a side of the case (41); the connecting sleeve segment (411) is detachably sleeved on the piston cylinder (221) of the conversion mechanism (20); the driving unit (42) is mounted in the connecting sleeve segment (411) and is capable of moving linearly;
the driving mechanism (40) has an electrical driving unit (42) and a controlling unit (44); the electrical driving unit (42) is mounted in the case (41); the electrical driving unit (42) has a driving assembly (431) and a transmission assembly (432); the transmission assembly (432) is connected to the driving assembly (431) and the driving unit (42); the controlling unit (44) is mounted in the case (41); the controlling unit (44) has a controlling circuit board (441) and a display panel (442) electrically connected to the controlling circuit board (441); the display panel (442) is exposed outside the case (41).

8. The injector as claimed in of claim 7, and **characterized in that** the transmission assembly (432) has a transmission rod (4321) and a gear assembly (4322); the transmission rod (4321) is pivotally mounted in the case (41); the transmission rod (4321) has at least one eccentric cam (4323); the gear assembly (4322) is connected between the driving assembly (431) and the transmission rod (4321); the eccentric cam (4323) of the transmission rod (4321) is connected to the driving unit (42); a restoring elastic unit (344) is mounted between the driving unit (42) and the case (41).

9. The injector as claimed in of claim 7, and **characterized in that** the driving mechanism (40) has a power storage unit (45); the power storage unit (45) is mounted in the case (41) and is electrically connected to the controlling circuit board (441) of the controlling unit (44).

## Patentansprüche

1. - Injektor, umfassend:
ein Medikamentbehälter (10), der einen das Medikament aufnehmenden Behälterhauptkörper (11) und einen an einer Seite des Behälterhauptkörpers (11) montierten Behälterverbindungsanschluss (12) aufweist;
ein Injektionsmodul (2), das Folgendes aufweist
einen Umwandlungsmechanismus (20), der mit dem Medikamentbehälter (10) verbunden ist; wobei der Umwandlungsmechanismus (20) eine Umwandlungsverbindungsbasis (21) und einen in der Umwandlungsverbindungsbasis (21) montierten Antriebskolbensatz (22) aufweist; die Umwandlungsverbindungsbasis (21) einen Eingangsadapter (213) und einen Ausgangsadapter (214) aufweist; die Umwandlungsverbindungsbasis (21) über den Eingangsadapter (213) mit dem Behälterverbindungsanschluss (12) des Medikamentbehälters (10) verbunden ist; und
einen Nadelausgangsmechanismus (30), der mit dem Umwandlungsmechanismus (20) verbunden ist; wobei der Nadelausgangsmechanismus (30) einen Nadelsatz (33) aufweist; wobei der Nadelsatz (33) angepasst ist, um mit dem Ausgangsadapter (214) verbunden zu werden; und
einen Antriebsmechanismus (40), der lösbar mit dem Umwandlungsmechanismus (20) des Injektionsmoduls (2) verbunden ist; wobei der Antriebsmechanismus (40) ein Gehäuse (41) und eine Antriebseinheit (42), die in dem Gehäuse (41) montiert und angepasst ist, um zur Bewegung betätigt zu werden; das Gehäuse (41) lösbar mit der Umwandlungsverbindungsbasis (21) des Umwandlungsmechanismus (20) verbunden ist; die Antriebseinheit (42) mit dem Antriebskolbensatz (22) verbunden und in der Lage ist, den Antriebskolbensatz (22) anzutreiben, um das Medikament aus dem Medikamentbehälter (10) anzusaugen und zu dem Nadelausgangsmechanismus (30) zu transportieren, und dann das Medikament mit dem Nadelsatz (33) des Nadelausgangsmechanismus (30) zu injizieren;
wobei die Injektionsvorrichtung, **dadurch gekennzeichnet ist, dass** der Nadelausgangsmechanismus (30) Folgendes aufweist:
eine Basis (31), die mit der Umwandlungsverbindungsbasis (21) verbunden ist;
ein Verbindungselement (32), das bewegbar in der Basis (31) montiert und mit dem Ausgangsadapter (214) des Umwandlungsmechanismus (20) verbunden ist, wobei der Nadelsatz (33) bewegbar in dem Verbindungselement (32) montiert ist und eine weiche Nadel (331), einen Nadelstopfen (332), der an einem Ende der weichen Nadel (331) montiert ist, und eine harte Nadel (333), die in der Lage ist, in der weichen Nadel (331) montiert zu werden, aufweist;
eine Nadelausgang-Betätigungseinheit (34), die bewegbar in der Basis (31) montiert und mit dem Nadelsatz (33) verbunden ist; wobei die Nadelausgang-Betätigungseinheit (34) angepasst ist, um betätigt zu werden, um den Nadelsatz (33), der sich in dem Verbindungselement (32) befindet anzutreiben, um mit der harten Nadel (333), die in der weichen Nadel (331) montiert ist, aus der Basis (31) hervorzustehen und dann die harte Nadel (333) anzutreiben, um sich von der weichen Nadel (331) zu lösen, damit die weiche Nadel (331) über das Verbindungselement (32) mit dem Ausgangsadapter (214) des Umwandlungsmechanismus (20) verbunden wird; und
eine Nadelrückzug-Betätigungseinheit (35), die bewegbar in der Basis (31) montiert ist; wobei die Nadelrückzug-Betätigungseinheit (35) angepasst ist, um betätigt zu werden, um das Verbindungselement (32) und die in dem Verbindungselement (32) montierte weiche Nadel (331) anzutreiben, um sich zu der Basis (31) zurückzuziehen.

2. - Injektor nach Anspruch 1, und **dadurch gekennzeichnet, dass** die Basis (31) des Nadelausgangsmechanismus (30) einen Aktionsraum (311) aufweist; eine Rückseite der Basis (31) eine Platte der Basis (31) und ein Eindringloch (313), das an der Platte der Basis (31) gebildet und mit dem Aktionsraum (311) verbunden ist, aufweist; eine Seite die dem Umwandlungsmechanismus (20) zugewandt ist, der Basis (31), eine mit dem Aktionsraum (311) verbundene Aktionsöffnung (315) aufweist; und ein Befestigungsrasthaken (2132) an der Basis (31) montiert ist und sich außerhalb des Aktionsraums (311) befindet;
das Verbindungselement (32) bewegbar in dem Aktionsraum (311) der Basis (31) montiert ist; das Verbindungselement (32) ein Verbindungssegment der Basis (31) und einen Verbindungskopf (322) aufweist; das Verbindungssegment der Basis (31) eine ringförmige Wand der Basis (31) und ein Montagesegment (3212) des Nadelsatzes (33), das in der ringförmigen Wand der Basis (31) montiert ist, aufweist; das Montagesegment (3212) des Nadelsatzes (33) einen in dem Montagesegment (3212) des Nadelsatzes (33) gebildeten Nadelraum (3213) und ein Loch (3214) des Nadelstopfens (332), das an einem Ende, das der Platte der Basis (31) zugewandt ist, des Montagesegments (3212) des Nadelsatzes (33) gebildet ist und mit dem Nadelraum (3213) verbunden ist, aufweist; das Loch (3214) des Nadelstopfens (332) dem Eindringloch (313) der Platte der Basis (31) entspricht; der Verbindungskopf (322) auf der Verbindungsbasis (31) montiert und mit dem Nadelraum (3213) in dem Montagesegment (3212) des Nadelsatzes (33) verbunden ist; der Verbindungskopf (322) durch die Aktionsöffnung (315) der Basis (31) montiert und mit dem Ausgangsadapter (214) des Umwandlungsmechanismus (20) verbunden ist; die ringförmige Wand der Basis (31) mindestens ein Schneidklingensegment (3215) aufweist;
die weiche Nadel (331) des Nadelsatzes (33) bewegbar in dem Nadelraum (3213) des Montagesegments (3212) des Nadelsatzes (33) montiert ist; der Nadelstopfen (332), der an der weichen Nadel (331) montiert ist, in der Lage ist, in dem Loch (3214) des Nadelstopfens (332) befestigt zu werden; und
die Nadelausgang-Betätigungseinheit (34) eine Nadelausgang-Betätigungseinheit (341), eine Nadelbasis (31), eine Dichtungseinheit (343) und eine elastische Einheit (344) aufweist; die Nadelausgang-Betätigungseinheit (341) bewegbar in der Basis (31) montiert ist und die Nadelausgang-Betätigungseinheit (341) in der Lage ist, durch den Befestigungsrasthaken (2132) der Basis (31) befestigt und eingerastet zu werden; die Nadelausgang-Betätigungseinheit (341) ein Tastensegment (3411) und einen Aufnahmeraum, der sich an einer Seite des Tastensegments (3411) befindet, aufweist; der Aufnahmeraum dem Aktionsraum (311) entspricht; sich die Nadelbasis (31) in dem Aufnahmeraum der Nadelausgang-Betätigungseinheit (341) befindet und mit der Nadelausgang-Betätigungseinheit (341) verbunden ist; die Nadelbasis (31) ein Kopfende der Nadelbasis (31) und eine Verbindungsstange der Nadelbasis (31), die an dem Kopfende der Nadelbasis (31) gebildet ist, aufweist; ein Verbindungsabschnitt (3423) zwischen einem Umfang des Kopfendes der Nadelbasis (31) und einer Innenwand des Aufnahmeraums der Nadelausgang-Betätigungseinheit (341) montiert und in der Lage ist, durch das Schneidklingensegment (3215) durchtrennt zu werden; die Verbindungsstange der Nadelbasis (31) mit der harten Nadel (333) verbunden ist und in der Lage ist, den Nadelstopfen (332) des Nadelsatzes (33) zu verschieben; sich die mit der elastischen Einheit (344) zusammengesetzte Dichtungseinheit (343) in dem Verbindungselement (32) befindet; sich die Dichtungseinheit (343) außerhalb des Montagesegments (3212) des Nadelsatzes (33) befindet und den Nadelraum (3213) abdeckt; die Dichtungseinheit (343) fest auf eine Außenseite der Verbindungsstange des Nadelbasis (31) aufgesteckt ist; die elastische Einheit (344) auf die Außenseite der Verbindungsstange des Nadelbodens (31) aufgesteckt ist; zwei Enden der elastischen Einheit (344) jeweils mit dem Kopfende der Nadelbasis (31) der Nadelbasis (31) und der Dichtungseinheit (343) verbunden sind.

3. - Injektor nach Anspruch 2, und **dadurch gekennzeichnet, dass** ein Durchmesserreduzierungssegment (3221) an einem Ende, das mit dem Segment der Verbindungsbasis (31) verbunden ist, des Verbindungskopfs (322) des Verbindungselements (32) gebildet ist, mit einem reduzierten Durchmesser gebildet ist und in der Lage ist, abgetrennt zu werden; ein Schneidmessersegment (3412) an einer Seite, die dem Verbindungskopf (322) entspricht, der Nadelausgang-Betätigungseinheit (341), montiert ist; das Schneidmessersegment (3412) in der Lage ist, betätigt zu werden, um das Durchmesserreduzierungssegment (3221) des Verbindungskopfs (322) des Verbindungselements (32) abzutrennen.

4. - Injektor nach Anspruch 2, und **dadurch gekennzeichnet, dass** ein Durchgangsloch (314) an einer Seite, die angrenzend an den Aktionsraum (311) ist, der Basis (31) gebildet ist und mit dem Aktionsraum (311) verbunden ist; sich die Nadelrückzug-Betätigungseinheit (35) von einer Außenseite der Basis (31) über das Durchgangsloch (314) in den Aktionsraum (311) erstreckt; die Nadelrückzug-Betätigungseinheit (35) ein Anpresssegment (351), eine anliegende geneigte Fläche (352) und ein Verlängerungssegment (353) aufweist; sich das Anpresssegment (351) bis zu der Außenseite der Basis (31) erstreckt; sich die anliegende geneigte Fläche (352) an einer Seite, die sich in den Aktionsraum (311) erstreckt, des Anpresssegments (351) befindet; die anliegende geneigte Fläche (352) an der ringförmigen Wand der Basis (31) des Verbindungselements (32) anliegt; sich das Verlängerungssegment (353) an der Seite, die sich in den Aktionsraum (311) erstreckt, des Anpresssegments (351) befindet und das Montagesegment (3212) des Nadelsatzes (33) des Verbindungselements (32) umgeht;
ein Durchmesserreduzierungssegment (3221) an einem Ende, das mit dem Segment der Verbindungsbasis (31) verbunden ist, des Verbindungskopfs (322) des Verbindungselements (32) gebildet ist, mit einem reduzierten Durchmesser gebildet ist und in der Lage ist, abgetrennt zu werden; ein Schneidmessersegment (3412) an einer Seite, die dem Verbindungskopf (322) entspricht, der Nadelausgang-Betätigungseinheit (341), montiert ist; das Schneidmessersegment (3412) in der Lage ist, betätigt zu werden, um das Durchmesserreduzierungssegment (3221) des Verbindungskopfs (322) des Verbindungselements (32) abzutrennen.

5. - Injektor nach einem der Ansprüche 1 bis 4, und **dadurch gekennzeichnet, dass** der Nadelausgangsmechanismus (30) ferner eine Schutzabdeckung (36) und einen Sicherungsbolzen (37) aufweist; die Schutzabdeckung (36) eine Außenseite der Nadelausgang-Betätigungseinheit (34) bedeckt und in der Lage ist, mit der Basis (31) einzugreifen; der Sicherungsbolzen (37) in der Sicherheitsabdeckung (36) und der Nadelausgang-Betätigungseinheit (341) der Nadelausgang-Betätigungsanordnung (34) montiert ist; eine elastische Verschiebeeinheit (344) zwischen der Schutzabdeckung (36) und der Nadelausgang-Betätigungseinheit (341) montiert ist.

6. - Injektor nach einem der Ansprüche 1 bis 4, und **dadurch gekennzeichnet, dass** die Umwandlungsverbindungsbasis (21) einen Körper (211) der Basis (31) und ein Segment (212) einer Basis (31) eines Ventils (222), das an dem Körper (211) der Basis (31) montiert ist, aufweist; das Segment (212) der Basis (31) des Ventils (222) eine Hülsenöffnung aufweist; der Eingangsadapter (213) an dem Segment (212) der Basis (31) des Ventils (222) montiert ist und sich auf einer Seite gegenüber der Hülsenöffnung befindet; der Ausgangsadapter (214) in dem Körper (211) der Basis (31) montiert ist; der Eingangsadapter (213) und der Ausgangsadapter (214) mit der Hülsenöffnung verbunden sind; der Antriebskolbensatz (22) einen Kolbenzylinder (221) aufweist, der einen Kolbenraum, ein Ventil (222) und eine Kolbenstange (223) aufweist, die in der Lage sind, sich linear in dem Kolbenzylinder (221) zu bewegen; der Kolbenzylinder (221) ein Ventil (222) verbindendes Endsegment (420) aufweist, das sich an einem Ende des Kolbenzylinders (221) befindet; das Ventil (222) verbindendes Endsegment (420) mit dem Ventil (222) zusammengesetzt und in dem Segment (212) der Basis (31) des Ventils (222) montiert ist; das Ventil (222) ein mit dem Eingangsadapter (213) verbundenes Einweg-Eingangsventil (222) und ein mit dem Ausgangsadapter (214) verbundenes Einweg-Ausgangsventil (222) aufweist; die Kolbenstange (223) in dem Kolbenzylinder (221) montiert und in der Lage ist, sich linear zu bewegen; die Kolbenstange (223) lösbar mit der Antriebseinheit (42) des Antriebsmechanismus (40) verbunden ist.

7. - Injektor nach Anspruch 6, und **dadurch gekennzeichnet, dass** das Gehäuse (41) ein Verbindungshülsensegment (411) aufweist, das sich auf einer Seite des Gehäuses (41) befindet; das Verbindungshülsenelement (411) lösbar auf den Kolbenzylinder (221) des Umwandlungsmechanismus (20) aufgesteckt ist; die Antriebseinheit (42) in dem Verbindungshülsenabschnitt (411) montiert und in der Lage ist, sich linear zu bewegen;
der Antriebsmechanismus (40) eine elektrische Antriebseinheit (42) und eine Steuereinheit (44) aufweist; die elektrische Antriebseinheit (42) in dem Gehäuse (41) montiert ist; die elektrische Antriebseinheit (42) eine Antriebsanordnung (431) und eine Übertragungsanordnung (432) aufweist; die Übertragungsanordnung (432) mit der Antriebsanordnung (431) und der Antriebseinheit (42) verbunden ist; die Steuereinheit (44) in dem Gehäuse (41) montiert ist; die Steuereinheit (44) eine Steuerplatine (441) und eine Anzeigetafel (442) aufweist, die elektrisch mit der Steuerplatine (441) verbunden ist; die Anzeigetafel (442) aus dem Gehäuse (41) hervorsteht.

8. - Injektor nach Anspruch 7, und **dadurch gekennzeichnet, dass** die Übertragungsanordnung (432) eine Übertragungsstange (4321) und eine Zahnradanordnung (4322) aufweist; die Übertragungsstange (4321) schwenkbar in dem Gehäuse (41) montiert ist; die Übertragungsstange (4321) mindestens einen Exzenternocken (4323) aufweist; die Zahnradanordnung (4322) zwischen der Antriebsanordnung (431) und der Übertragungsstange (4321) verbunden ist; der Exzenternocken (4323) der Übertragungsstange (4321) mit der Antriebseinheit (42) verbunden ist; eine elastische Rückstelleinheit (344) zwischen der Antriebseinheit (42) und dem Gehäuse (41) montiert ist.

9. - Injektor nach Anspruch 7, und **dadurch gekennzeichnet, dass** der Antriebsmechanismus (40) eine Energiespeichereinheit (45) aufweist; die Energiespeichereinheit (45) in dem Gehäuse (41) montiert und elektrisch mit der Steuerplatine (441) der Steuereinheit (44) verbunden ist.

## Revendications

1. - Injecteur, comprenant :
un contenant à médicament (10) ayant un corps principal de contenant (11) recevant un médicament et un orifice de liaison de contenant (12) monté sur un côté du corps principal de contenant (11) ;
un module d'injection (2) ayant
un mécanisme de conversion (20) relié au contenant à médicament (10) ; le mécanisme de conversion (20) ayant une base de liaison de conversion (21) et un ensemble piston d'entraînement (22) monté dans la base de liaison de conversion (21) ; la base de liaison de conversion (21) ayant un adaptateur d'entrée (213) et un adaptateur de sortie (214) ; la base de liaison de conversion (21) étant reliée à l'orifice de liaison de contenant (12) du contenant à médicament (10) par l'intermédiaire de l'adaptateur d'entrée (213) ; et
un mécanisme de sortie d'aiguille (30) relié au mécanisme de conversion (20) ; le mécanisme de sortie d'aiguille (30) ayant un ensemble aiguille (33) ; l'ensemble aiguille (33) étant agencé pour être relié à l'adaptateur de sortie (214) ; et
un mécanisme d'entraînement (40) relié de manière détachable au mécanisme de conversion (20) du module d'injection (2) ; le mécanisme d'entraînement (40) ayant un boîtier (41) et une unité d'entraînement (42) montée dans le boîtier (41) et agencée pour être actionnée afin de se déplacer ; le boîtier (41) étant relié de manière détachable à la base de liaison de conversion (21) du mécanisme de conversion (20) ; l'unité d'entraînement (42) étant reliée à l'ensemble piston d'entraînement (22) et étant apte à entraîner l'ensemble piston d'entraînement (22) pour aspirer et transporter du médicament à partir du contenant à médicament (10) jusqu'au mécanisme de sortie d'aiguille (30), puis à injecter le médicament à l'aide de l'ensemble aiguille (33) du mécanisme de sortie d'aiguille (30) ;
l'injecteur étant **caractérisé par le fait que** le mécanisme de sortie d'aiguille (30) a :
une base (31) reliée à la base de liaison de conversion (21) ;
un élément de liaison (32) monté de manière mobile dans la base (31) et relié à l'adaptateur de sortie (214) du mécanisme de conversion (20), l'ensemble aiguille (33) étant monté de manière mobile dans l'élément de liaison (32) et ayant une aiguille souple (331), un embout d'aiguille (332) monté sur une extrémité de l'aiguille souple (331), et une aiguille rigide (333) apte à être montée dans l'aiguille souple (331) ;
un ensemble d'actionnement de sortie d'aiguille (34) monté de manière mobile dans la base (31) et relié à l'ensemble aiguille (33) ; l'ensemble d'actionnement de sortie d'aiguille (34) étant agencé pour être actionné afin d'entraîner l'ensemble aiguille (33) situé dans l'élément de liaison (32) pour qu'il fasse saillie hors de la base (31) avec l'aiguille rigide (333) montée dans l'aiguille souple (331), puis d'entraîner l'aiguille rigide (333) pour qu'elle se détache de l'aiguille souple (331) afin de permettre à l'aiguille souple (331) d'être reliée à l'adaptateur de sortie (214) du mécanisme de conversion (20) par l'intermédiaire de l'élément de liaison (32) ; et
une unité d'actionnement de retour d'aiguille (35) montée de manière mobile dans la base (31) ; l'unité d'actionnement de retour d'aiguille (35) étant agencée pour être actionnée afin d'entraîner l'élément de liaison (32) et l'aiguille souple (331) montée dans l'élément de liaison (32) à se rétracter vers la base (31).

2. - Injecteur selon la revendication 1, **caractérisé par le fait que** la base (31) du mécanisme de sortie d'aiguille (30) a une chambre d'action (311) ; une surface arrière de la base (31) a une plaque de base (31) et un trou de pénétration (313) formé sur la plaque de base (31) et relié à la chambre d'action (311) ; un côté de la base (31), qui fait face au mécanisme de conversion (20), a une ouverture d'action (315) reliée à la chambre d'action (311) ; et un crochet de fixation à enclenchement (2132) est monté sur la base (31) et situé à l'extérieur de la chambre d'action (311) ;
l'élément de liaison (32) est monté de manière mobile dans la chambre d'action (311) de la base (31) ; l'élément de liaison (32) a un segment de base de liaison (31) et une tête de liaison (322) ; le segment de base de liaison (31) a une paroi annulaire de base (31) et un segment de montage (3212) d'ensemble aiguille (33) monté dans la paroi annulaire de base (31) ; le segment de montage (3212) d'ensemble aiguille (33) a une chambre d'aiguille (3213) formée dans le segment de montage (3212) d'ensemble aiguille (33) et un trou (3214) d'embout d'aiguille (332) formé sur une extrémité, qui fait face à la plaque de base (31), du segment de montage (3212) d'ensemble aiguille (33) et relié à la chambre d'aiguille (3213) ; le trou (3214) d'embout d'aiguille (332) correspond au trou de pénétration (313) de la plaque de base (31) ; la tête de liaison (322) est montée sur la base de liaison (31) et reliée à la chambre d'aiguille (3213) dans le segment de montage (3212) d'ensemble aiguille (33) ; la tête de liaison (322) est montée à travers l'ouverture d'action (315) de la base (31) et est reliée à l'adaptateur de sortie (214) du mécanisme de conversion (20) ; la paroi annulaire de base (31) a au moins un segment de lame de coupe (3215) ;
l'aiguille souple (331) de l'ensemble aiguille (33) est montée de manière mobile dans la chambre d'aiguille (3213) du segment de montage (3212) d'ensemble aiguille (33) ; l'embout d'aiguille (332) qui est monté sur l'aiguille souple (331) est apte à être fixé dans le trou (3214) d'embout d'aiguille (332) ; et
l'ensemble d'actionnement de sortie d'aiguille (34) a une unité d'actionnement de sortie d'aiguille (341), une base d'aiguille (31), une unité d'étanchéité (343) et une unité élastique (344) ; l'unité d'actionnement de sortie d'aiguille (341) est montée de manière mobile dans la base (31), et l'unité d'actionnement de sortie d'aiguille (341) est apte à être fixée et enclenchée par le crochet de fixation à enclenchement (2132) de la base (31) ; l'unité d'actionnement de sortie d'aiguille (341) ayant un segment de bouton (3411) et une chambre de réception située sur un côté du segment de bouton (3411) ; la chambre de réception correspond à la chambre d'action (311) ; la base d'aiguille (31) est située dans la chambre de réception de l'unité d'actionnement de sortie d'aiguille (341) et est reliée à l'unité d'actionnement de sortie d'aiguille (341) ; la base d'aiguille (31) a une extrémité de tête de base d'aiguille (31) et une tige de liaison de base d'aiguille (31) formée sur l'extrémité de tête de base d'aiguille (31) ; un segment de liaison (3423) est monté entre une périphérie de l'extrémité de tête de base d'aiguille (31) et une paroi interne de la chambre de réception de l'unité d'actionnement de sortie d'aiguille (341) et est apte à être coupé par le segment de lame de coupe (3215) ; la tige de liaison de base d'aiguille (31) est reliée à l'aiguille rigide (333) et est apte à pousser l'embout d'aiguille (332) de l'ensemble aiguille (33) ; l'unité d'étanchéité (343) assemblée à l'unité élastique (344) est située dans l'élément de liaison (32) ; l'unité d'étanchéité (343) est située à l'extérieur du segment de montage (3212) d'ensemble aiguille (33) et recouvre la chambre d'aiguille (3213) ; l'unité d'étanchéité (343) est emmanchée de manière étanche sur un côté extérieur de la tige de liaison de base d'aiguille (31) ; l'unité élastique (344) est emmanchée sur le côté extérieur de la tige de liaison de base d'aiguille (31) ; deux extrémités de l'unité élastique (344) sont respectivement reliées à l'extrémité de tête de base d'aiguille (31) de la base d'aiguille (31) et à l'unité d'étanchéité (343).

3. - Injecteur selon la revendication 2, **caractérisé par le fait qu'**un segment de réduction de diamètre (3221) est formé sur une extrémité, qui est reliée au segment de base de liaison (31), de la tête de liaison (322) de l'élément de liaison (32), comporte un diamètre réduit et est apte à être coupé ; un segment de couteau de coupe (3412) est monté sur un côté, qui correspond à la tête de liaison (322), de l'unité d'actionnement de sortie d'aiguille (341) ; le segment de couteau de coupe (3412) est apte à être actionné pour couper le segment de réduction de diamètre (3221) de la tête de liaison (322) de l'élément de liaison (32).

4. - Injecteur selon la revendication 2, **caractérisé par le fait qu'**un trou traversant (314) est formé sur un côté, qui est adjacent à la chambre d'action (311), de la base (31) et est relié à la chambre d'action (311) ; l'unité d'actionnement de retour d'aiguille (35) s'étend dans la chambre d'action (311) à partir d'un côté extérieur de la base (31) par l'intermédiaire du trou traversant (314) ; l'unité d'actionnement de retour d'aiguille (35) a un segment de pression (351), une surface inclinée de butée (352) et un segment d'extension (353) ; le segment de pression (351) s'étend jusqu'au côté extérieur de la base (31) ; la surface inclinée de butée (352) est située sur un côté, qui s'étend dans la chambre d'action (311), du segment de pression (351) ; la surface inclinée de butée (352) vient en butée contre la paroi annulaire de base (31) de l'élément de liaison (32) ; le segment d'extension (353) est situé sur le côté, qui s'étend dans la chambre d'action (311), du segment de pression (351) et contourne le segment de montage (3212) d'ensemble aiguille (33) de l'élément de liaison (32) ;
un segment de réduction de diamètre (3221) est formé sur une extrémité, qui est reliée au segment de base de liaison (31), de la tête de liaison (322) de l'élément de liaison (32), comporte un diamètre réduit et est apte à être coupé ; un segment de couteau de coupe (3412) est monté sur un côté, qui correspond à la tête de liaison (322), de l'unité d'actionnement de sortie d'aiguille (341) ; le segment de couteau de coupe (3412) est apte à être actionné pour couper le segment de réduction de diamètre (3221) de la tête de liaison (322) de l'élément de liaison (32).

5. - Injecteur selon l'une quelconque des revendications 1 à 4, **caractérisé par le fait que** le mécanisme de sortie d'aiguille (30) a en outre un capot de sécurité (36) et un verrou de sécurité (37) ; le capot de sécurité (36) recouvre un côté extérieur de l'ensemble d'actionnement de sortie d'aiguille (34) et est apte à s'engager avec la base (31) ; le verrou de sécurité (37) est monté dans le capot de sécurité (36) et l'unité d'actionnement de sortie d'aiguille (341) de l'ensemble d'actionnement de sortie d'aiguille (34) ; une unité élastique de poussée (344) est montée entre le capot de sécurité (36) et l'unité d'actionnement de sortie d'aiguille (341).

6. - Injecteur selon l'une quelconque des revendications 1 à 4, **caractérisé par le fait que** la base de liaison de conversion (21) a un corps (211) de base (31) et un segment (212) de base (31) de valve (222) monté sur le corps (211) de base (31) ; le segment (212) de base (31) de valve (222) a une ouverture de manchon ; l'adaptateur d'entrée (213) est monté sur le segment (212) de base (31) de valve (222) et est situé sur un côté opposé à l'ouverture de manchon ; l'adaptateur de sortie (214) est monté dans le corps (211) de base (31) ; l'adaptateur d'entrée (213) et l'adaptateur de sortie (214) sont reliés à l'ouverture de manchon ; l'ensemble piston d'entraînement (22) a un cylindre de piston (221) ayant une chambre de piston, une valve (222), et une tige de piston (223) apte à se déplacer linéairement dans le cylindre de piston (221) ; le cylindre de piston (221) a un segment d'extrémité de liaison (420) de valve (222) situé sur une extrémité du cylindre de piston (221) ; le segment d'extrémité de liaison (420) de valve (222) est assemblé à la valve (222) et est monté dans le segment (212) de base (31) de valve (222) ; la valve (222) a un clapet de non-retour d'entrée (222) relié à l'adaptateur d'entrée (213) et un clapet de non-retour de sortie (222) relié à l'adaptateur de sortie (214) ; la tige de piston (223) est montée dans le cylindre de piston (221) et est apte à se déplacer linéairement ; la tige de piston (223) est reliée de manière détachable à l'unité d'entraînement (42) du mécanisme d'entraînement (40).

7. - Injecteur selon la revendication 6, **caractérisé par le fait que** le boîtier (41) a un segment de manchon de liaison (411) situé sur un côté du boîtier (41) ; le segment de manchon de liaison (411) est emmanché de manière détachable sur le cylindre de piston (221) du mécanisme de conversion (20) ; l'unité d'entraînement (42) est montée dans le segment de manchon de liaison (411) et est apte à se déplacer linéairement ;
le mécanisme d'entraînement (40) a une unité d'entraînement électrique (42) et une unité de commande (44) ; l'unité d'entraînement électrique (42) est montée dans le boîtier (41) ; l'unité d'entraînement électrique (42) a un ensemble d'entraînement (431) et un ensemble de transmission (432) ; l'ensemble de transmission (432) est relié à l'ensemble d'entraînement (431) et à l'unité d'entraînement (42) ; l'unité de commande (44) est montée dans le boîtier (41) ; l'unité de commande (44) a une carte de circuit de commande (441) et un panneau d'affichage (442) relié électriquement à la carte de circuit de commande (441) ; le panneau d'affichage (442) est exposé à l'extérieur du boîtier (41).

8. - Injecteur selon la revendication 7, **caractérisé par le fait que** l'ensemble de transmission (432) a une tige de transmission (4321) et un ensemble engrenage (4322) ; la tige de transmission (4321) est montée de manière pivotante dans le boîtier (41) ; la tige de transmission (4321) a au moins une came excentrique (4323) ; l'ensemble engrenage (4322) est relié entre l'ensemble d'entraînement (431) et la tige de transmission (4321) ; la came excentrique (4323) de la tige de transmission (4321) est reliée à l'unité d'entraînement (42) ; une unité élastique de rappel (344) est montée entre l'unité d'entraînement (42) et le boîtier (41).

9. - Injecteur selon la revendication 7, **caractérisé par le fait que** le mécanisme d'entraînement (40) a une unité d'accumulation d'énergie (45) ; l'unité d'accumulation d'énergie (45) est montée dans le boîtier (41) et est reliée électriquement à la carte de circuit de commande (441) de l'unité de commande (44).
